# EUROPEAN PATENT APPLICATION

(11) **EP 4 162 872 A1**
(43) Date of publication of application: **12.04.2023**
(21) Application number: 21201079.7
(22) Date of filing: 05.10.2021
(51) Int. Cl.: A61B 5/12, A61B 5/00

(54) **A COMPUTER-IMPLEMENTED METHOD FOR OPTIMIZING A HEARING TEST FOR ESTIMATING PURE TONE HEARING THRESHOLDS OF A SUBJECT**

(71) Applicant: Binary Perception P.C., 15344 Gerakas Attiki (GR)
(72) Inventor: Tsitsanis, Evangelos, Gerakas (GR); Ntalkos, Lampros, Gerakas (GR)
(74) Representative: Kouzelis, Dimitrios

(57) **Abstract**

The present invention refers to a computer-implemented method for optimizing a hearing test for estimating pure tone hearing thresholds of a subject. The method comprises the step of determining a plurality of test points (t_{s,f}), wherein each test point (t_{s,f}) is defined by a frequency value and information about the ear side to be tested, the step of selecting a test point (t_{s,f}) of the plurality of test points (t_{s,f}) in a random way, the step of assigning an amplitude level to the selected test point (t_{s,f}), the step of generating a signal that triggers the generation of an audio signal based on the selected test point (t_{s,f}) and assigned amplitude level for representing a corresponding acoustic stimulus, and the step of detecting a response of the subject to the presentation of the acoustic stimulus to the subject.

## Description

The present invention generally refers to hearing tests, which are used for evaluating the sensitivity of a person's sense of hearing. The sensitivity of a person's hearing is expressed by the so-called hearing thresholds, each of which corresponds to the sound level below which a subject's ear is unable to detect a sound of a specific frequency.

In particular, the present invention refers to a computer-implemented method for optimizing a hearing test for estimating pure tone hearing thresholds of a subject.

A hearing test is most often performed by a professional ENT doctor or an audiologist using an audiometer for determining a person's hearing sensitivity at different frequencies. Pure Tone Audiometry (PTA), usually referred to as the "golden standard of audiometry", is still the most common hearing test procedure that professional ENT doctors and audiologists around the globe use to identify the hearing thresholds in a chosen set of frequencies of an individual. It results in an audiogram that shows the type (conductive, sensorineural or mixed) and level of hearing loss. The latest and most commonly used version of PTA procedure is the modified Hughson-Westlake procedure.

From a physical point of view, a sound is a vibration that typically propagates as an audible wave of pressure through a transmission medium such as a gas, liquid or solid. Although many complexities relating to the transmission of sound exist, sound is basically made of two elements: pressure and time. These two elements alone could describe, in absolute terms, all characteristics of a sound just before it enters a person's ear. However, describing the physical properties of a sound is totally different from what its mental imprint on a person is, i.e. the perception of sound.

Principally, for understanding how sounds are perceived, sound needs to be disassembled to its primary components. To this end, sound can be simplified as a multiplication of a plethora of sinusoidal tones of different frequencies and intensities, namely as a multiplication of pure tones. The perceptual correlate of a sound's intensity is called "loudness", a term that usually describes how loud a sound is. Likewise, the perceptual correlate of a sound's frequency is called pitch. Other things being equal, an increase or decrease in either intensity or frequency of a tone (of a sound wave coming in) is perceived as an increase or decrease in loudness or pitch, respectively.

It is apparent that, in order to acquire a more detailed mirroring of the basic physical properties of intensity and frequency of sound to its perceptual counterparts, a measurement of the latter is inevitable.

Historically, the righteous determination of the hearing thresholds has been investigated by psychoacoustic measurements. In particular, different classical and adaptive psychoacoustic methods have been used.

Known methods comprise e.g. the method of limits, the method of adjustment, the method of stimuli, Bekesy's tracking method, the simple "up-and-down" method, the PEST adaptive method, the transformed "up-and-down" method and the block "up-and-down" method. These methods make use of the "Yes/No" approach, according to which the subject is asked to react only in case he/she perceives the acoustic stimulus presented and do nothing if he/she does not perceive anything. On the other hand, the so-called "forced choice methodologies", which can also be used in combination with the main steps of all the aforementioned methods, require the subject's reaction not only when the subject perceives the presented acoustic stimulus, but also when the subject does not perceive it. This approach involves acoustic stimulus presentations, with two or more alternatives in successive intervals, from which the subject must choose which interval contains the acoustic stimulus, i.e. the "correct" one. The most recent psychoacoustic methodologies introduce adaptive procedures in combination with maximum likelihood estimation and/or Bayesian theory in their paradigm.

Although all the aforementioned psychoacoustic methods establish relationships between the physical properties of a tone and its perceptual correlates (loudness and pitch), they all accomplish it in different ways, thereby resulting in methods of different quality. Quality can in general be defined by the characteristics of speed, reliability and accuracy. Speed is a crucial factor, since measurements of longer duration tend to result in fatigue artifacts caused by fatigue of the subject. Reliability, on the other hand, is a measure of the reproducibility of the measurement. It is performed as a test-retest procedure within a specific time interval as a measure of consistency of the results. In mathematical terms, it is usually calculated through a mean square error formula. Test - retest standard deviation, in industrial audiometry, may vary between 6 dB to 10 dB, but these results do not reflect all population hearing profiles. While speed and reliability are two quality factors that can easily be computed, accuracy is a quality property that is poorly defined as it fails to point out in a more objective way which psychophysical methodology best represents "true" auditory threshold estimation and why.

It is an object underlying the present invention to provide a computer-implemented method for optimizing a hearing test for estimating pure tone hearing thresholds of a subject.

Said object is achieved by a computer-implemented method for optimizing a hearing test for estimating pure tone hearing thresholds of a subject. The method comprises the steps of determining a plurality of test points, selecting a test point of the plurality of test points, assigning an amplitude level to the selected test point, generating a signal for triggering the generation of an audio signal based on the selected test point and assigned amplitude level for representing a corresponding acoustic stimulus, and detecting a response of the subject to the presentation of the acoustic stimulus to the subject. Each test point is defined by a frequency value and information about the ear side to be tested.

The step of generating the triggering signal can in particular also be formulated as generating a signal for triggering, for the selected test point and assigned amplitude level, the generation of an audio signal based on the selected test point and assigned amplitude level, for representing a corresponding acoustic stimulus.

An acoustic stimulus is in particular defined as the presentation of a test point of the plurality of test points at an amplitude level to the subject. Thus, the acoustic stimulus is in the present invention advantageously defined by the selected test point and the amplitude level assigned to it.

The formulation "for representing a corresponding acoustic stimulus" means in particular that the audio signal is configured to cause, if input to a sound reproducing means, the sound reproducing means to produce an acoustic stimulus defined by the selected test point and the assigned amplitude level.

Advantageously, the selection of the test point in the aforementioned selecting step is done in a random way (order). The random selection of a test point means that the test point to be presented to the subject is not selected by following a predetermined order. In particular, this means that the selection of the test points does not follow a predetermined order with regard to the frequency and ear side. It is further understood that the random selection of the test point advantageously means that the corresponding acoustic stimulus will be/is presented to the subject in a random order.

The aforementioned test point selecting step, signal generating step and response detecting step are advantageously repeated for all the test points of the plurality of test points. This means that each test point is advantageously selected in a random way out of the plurality of the test points and a corresponding amplitude level is assigned thereto. A corresponding signal that triggers the generation of an audio signal based on the corresponding selected test point and assigned amplitude level for representing a corresponding acoustic stimulus is generated for each selected test point and the corresponding assigned amplitude level, and a corresponding response of the subject to the presentation of the corresponding acoustic stimulus to the subject is detected.

Due to the random selection of the test point(s), and thus also the random presentation of the corresponding acoustic stimulus/stimuli to the subject, the proposed method has the advantage that it reduces the bias that would otherwise be caused in the case of a selection of the test points and thus also of the presentation of the acoustic stimuli to the subject in a predetermined order in terms of their frequency and/or their amplitude and/or tested ear side. The reason is that in the case of the presentation of the acoustic stimuli to the subject in a predetermined order in terms of their frequency and/or their amplitude and/or tested ear side, the subject would eventually realize the pattern with which the acoustic stimuli are presented to him/her. This would in turn affect his/her responses to the acoustic stimuli, as he/she would most probably be able to guess which acoustic stimulus would be presented next. For example, if the predetermined order consists in presenting the plurality of test points in an ascending order in view of their frequencies, the subject would soon realize that the next test point to be presented should/would have a higher frequency than the previously presented point. Thus, the subject could be influenced by this "finding" in his/her response. This effect would be more pronounced in the case where many test points were presented to the subject, as the subject would have enough time to realize the pattern of the presentation of the test points. In another example, current audiometers, according to ISO-8253-1 standard, estimate each test point's threshold before continuing to the next one. For each threshold estimation of a specific test point, they use a predetermined series of either ascending or descending amplitude runs. As a consequence, they suffer from the 'phenomenon of anticipation', in which the subject becomes quickly aware of the fact that the ascending amplitude runs come just after the descending amplitude runs, hence the subject is prepared and falsely pre-convinced to hear them. This results in better 'ascending' thresholds. 'Phenomenon of perceptual hysteresis' is the phenomenon in which the subject during the descending amplitude runs knows that the next quieter tone always follows, hence he/she is more motivated to hear it. This falsely produces better 'descending' thresholds. Lastly, the 'phenomenon of habituation' (as the opposite of the 'phenomenon of anticipation') is responsible for the fact that the subject becomes accustomed to responding either positively in the descending runs and/or not reacting (waiting for the next louder sound) to the ascending ones. On the contrary, the randomized order of the generation of the electric signals corresponding to acoustic stimuli and thus also the randomized presentation of the acoustic stimuli to the subject makes sure that the subject will not be able to guess the subsequent acoustic stimulus that will presented to him/her. In other words, subjects will be unaware of the ear side and the frequency, and/or the amplitude, on which they are going to be tested next. Thus, many test points can be presented to the subject during the hearing test without the risk of an increased bias. This results in a least-biased method for optimizing a hearing test for estimating pure tone hearing thresholds of a subject.

It becomes apparent that the amplitude level assigned to a selected test point corresponds to the presentation level of this test point, i.e. the amplitude level at which the test point is presented to the subject or the amplitude level of the corresponding acoustic stimulus. It further becomes apparent that the step of assigning an amplitude level to a selected test point advantageously follows the selection of the test point. This means that a test point is first selected and then an amplitude level at which the test point will be presented to the subject is assigned to the test point.

It is noted that within the scope of the present invention, the expressions "response of a subject to the presentation of a test point at an amplitude level or "response of a subject to the presentation of an acoustic stimulus" more particularly refer to both the case where the subject has perceived the acoustic stimulus, i.e. has heard the sound presented to him/her, and the case where the subject has not perceived the acoustic stimulus, i.e. has not heard the sound presented to him/her. In the former case, it is particularly said that the response of the subject is a positive response, while in the latter case the response of the subject is particularly characterized as a negative response. A positive response can preferably be given by the subject by actively giving his/her response, e.g. by acting on a response input means. A negative response can preferably be in the form of no response, i.e. when the subject does not act on said response input means. For example, if the response input means comprises a button, a positive response can be provided/detected, when the subject presses the button, while a negative response can be provided/detected, when the subject does not press the button.

Detecting a positive response preferably corresponds to receiving a signal indicating a positive response. Detecting a negative response preferably corresponds to receiving a signal indicating a negative response. In particular, in the case of no response to the presentation of a test point to the subject, detecting a response preferably corresponds to receiving no signal or receiving a signal indicating that no response was provided by the subject.

It is further noted that the information about the ear side to be tested indicates which ear side is to be tested and comprises the options "left ear side" and "right ear side".

In addition, it is noted that a pure tone is a sound with a sinusoidal waveform that is characterized by a frequency, a phase and an amplitude value.

The plurality of test points may more particularly comprise at least seven test points, preferably at least fifty test points, more preferably at least one hundred test points, in particular per ear side. For example, the plurality of test points may comprise fifty test points for the left ear side and fifty test points for the right ear side.

The method preferably comprises a step of generating the aforementioned audio signal.

The method advantageously comprises a step of estimating a hearing threshold for the selected test point, in particular a corresponding hearing threshold for each selected test point.

The estimation of hearing thresholds of a subject is advantageously done based on the responses of the subject to the presentation of the acoustic stimuli to him/her.

Within the framework of the present invention, the amplitude level at which a test point is presented to the subject indicates the loudness of the corresponding acoustic stimulus and is expressed in dB (decibels). The frequency of a test point is expressed in Hz or KHz.

Preferably, the combination of the selected test point and the assigned amplitude level is chosen in a semi-random order. This means that an acoustic stimulus based on the combination of a selected test point and the amplitude level assigned to it will not be presented a second time to the subject. Thus, the corresponding signal that triggers the generation of the corresponding audio signal and the audio signal will be generated only once. The word "semi" in the term "semi-random" more particularly means that every time a combination of a selected test point and its assigned amplitude level is chosen for generating a corresponding signal for triggering the generation of a corresponding audio signal cannot be chosen again. Thus, a generated signal is configured to trigger the generation of an audio signal based on a different combination of a selected test point and amplitude level assigned to it compared to the preceding generated audio signal. The different combination may refer to the same test point but with a different amplitude level or a different test point with an amplitude level assigned to it. In any case, the combination is randomly chosen. This means that even if the generated signal triggers the generation of an audio signal based on a selected test point, on which the preceding generated audio signal was also based, this will be the result of a random choice.

Thus, when an acoustic stimulus is presented to a subject, i.e. when a test point is presented to the subject at an amplitude level, the subject will not know which test point the next acoustic stimulus that will be presented to him/her will correspond to and/or at what amplitude level it will be presented. Even if a subsequent acoustic stimulus (different from the preceding acoustic stimulus) is based on a test point that has already been presented at an amplitude level to the subject, the subject will, due to the semi-randomness of the method, not be able to realize whether the subsequent presentation of the same test point is done at an amplitude level lower or higher than the amplitude level at which the test point has already been presented to the subject.

The method of the present invention advantageously comprises an evaluation phase, and in particular also an initialization phase, which can also be understood as an evaluation phase and an initialization phase of the hearing test, respectively. The initialization phase precedes the evaluation phase. The evaluation phase is advantageously the phase, during which the hearing threshold for each test point is estimated. The initialization phase is advantageously the phase, during which the test points are initialized.

Preferably, if the method comprises both an initialization phase and an evaluation phase, in the evaluation phase, the hearing threshold for each test point is estimated based on the results of the initialization phase. In particular, an amplitude level assigned to a selected test point is based on and is also different than an amplitude level assigned to the same test point in its preceding presentation taken place in the initialization phase depending on the response of the subject to its preceding presentation.

What preferably happens in the initialization phase will become apparent from the following description.

Preferably, during the initialization phase, each test point is selected only once. This means that during the initialization phase only one triggering signal is generated for each test point. This further means, that during the initialization phase, each test point is presented only once to the subject at a corresponding assigned amplitude level. The provision of the initialization phase is advantageous as it may provide an indication whether the corresponding amplitude level assigned to the selected test point and at which the selected test point is presented to the subject during the initialization phase, is a promising start for estimating the hearing threshold for the corresponding test point as quick as possible, i.e. with the least number of selections and thus presentations of the test point at different amplitude levels to the subject. In particular, the provision of the initialization phase is advantageous as it may provide a rough estimate of the hearing profile of a subject as quick as possible, i.e. for all test points. This estimation is valid, regardless of the subject's responses to the presentation of the test points in this phase. Further, the fact that all the test points are selected and also presented only once during the initialization phase is also advantageous for the reason that the estimation of whether the amplitude level at which a corresponding test point is presented to the subject is a good starting amplitude level for said test point for the subject being tested or not can be done without causing fatigue to the subject.

Preferably, each test point is associated with a corresponding initial amplitude level, in particular for the initialization phase, when the method comprises both an initialization phase and an evaluation phase, or the evaluation phase, when the method comprises only an evaluation phase.

The amplitude level assigned to a selected test point in the initialization phase can be equal to the corresponding initial amplitude level for this test point. In other words, each test point can be assigned with and presented at its corresponding initial amplitude level to the subject in the initialization phase.

However, according to an advantageous embodiment of the present invention, in the initialization phase, the amplitude level assigned to the first selected test point for each ear side is equal to its corresponding initial amplitude level. The amplitude level for any other selected test point of the plurality of test points is equal to an amplitude level, which is based on the initial amplitude level for the corresponding test point and the responses of the subject to the presentation of all test points, in particular for the same ear side with that of the corresponding test point, that have already been selected in the initialization phase. Thus, according to said advantageous embodiment of the present invention, in the initialization phase, the first test point for each ear side is presented to the subject at its corresponding initial amplitude level, and any other test point of the plurality of test points is presented to the subject at an amplitude level, which is based on the initial amplitude level for the corresponding test point and the responses of the subject to the presentation of all the already presented test points, in particular for the same ear side, in the initialization phase. This means that for determining the amplitude level, at which a test point other than the first test point for each ear side is presented in the initialization phase, the responses of the subject to the presentation of all test points, in particular for the same ear side, that have been presented so far in the initialization phase is/are taken into account. This has the advantages that, as the audiometric session progresses, the amplitude level of the remaining test points that are (soon) to be presented to the subject in the initialization phase, will be closer to the subject's hearing threshold for the corresponding test points.

Further, it is advantageous when the initial amplitude levels for the test points for estimating the hearing thresholds of a subject are based on data for subjects having the same age and/or gender and/or nationality, as it is probable that subjects having similar characteristics will have a similar hearing profile and thus the initial amplitude levels for the subject to be tested will probably be close to his/her hearing thresholds. Additionally or alternatively, the initial amplitude levels for the test points for estimating the hearing thresholds of a subject are advantageously based on data from any previous hearing tests of the same subject, as it may be the case that the previously estimated hearing thresholds for the subject have not changed or at least have not changed dramatically.

In any case, due to the above mentioned advantageous aspect of the present invention, the proposed method can quickly realize this aspect and adapt itself from the presentations of the test points in the initialization phase. Therefore, it does not "waste" selections and thus presentations of test points at amplitude levels that are probably quite far from the subject's hearing profile, what in turn results in a quicker determination of the subject's audibility and inaudibility boundaries.

It is noted that the term "first test point for each side" in the above formulation can be understood as the firstly presented test point for each side. Thus, the characterization of a test point as first test point does not imply anything on its frequency compared to the frequencies of the other test points of the plurality of test points. In particular, it does not imply that the first test point has the lowest frequency among the test points of the plurality of test points. Indeed, due to the random order in which the test points are selected and presented to the subject in the initialization phase, the first test point is randomly chosen and thus can be any test point within a portion of the audible range.

The above configuration can in particular be realized by assigning to each selected test point an amplitude level, which is determined based on the initial amplitude level for the corresponding test point and an adaptation amplitude offset for adapting said initial amplitude level. The adaptation amplitude offset for adapting the initial amplitude level of the first selected test point for each ear side is zero and the adaptation amplitude offset for any other test point is based on the responses of the subject to the presentation of all already selected test points, in particular for the same ear side, in the initialization phase. Thus, any test point of the plurality of test points is presented in the initialization phase at an amplitude level, which is determined based on the initial amplitude level for the corresponding test point and a corresponding adaptation amplitude offset for adapting said initial amplitude level.

Preferably, the adaptation amplitude offset for adapting the corresponding initial amplitude level of a test point other than the first selected test point is determined based on a second predetermined step for a corresponding test point and an offset parameter for the corresponding test point. The offset parameter used for determining the adaptation amplitude offset for the corresponding test point depends on the responses of the subject to the presentation of all the already presented test points, in particular for the same ear side, in the initialization phase. In other words, as the offset parameter for the corresponding test point depends on the responses of the subject to the presentation of all the already selected and thus presented test points, in particular for the same ear side, the offset parameter for determining the adaptation amplitude offset for the corresponding selected test point can be determined based on the offset parameter for determining the adaptation amplitude offset for the preceding selected test point and the response of the subject to the presentation of the preceding selected test point. In particular, the offset parameter for determining the adaptation amplitude offset for a corresponding selected test point may equal to the offset parameter for determining the adaptation amplitude offset for the preceding selected test point being increased or decreased, in particular by one, depending on the response of the subject to the presentation of the preceding selected test point. If the response is a positive response, the offset parameter is decreased. If the response is a negative response, the offset parameter is increased.

Within the context of the present invention, the offset parameter can also be described as LEFT_OFFSET, when referring to the left ear side test points, and as RIGHT_OFFSET, when referring to the right ear side test points.

As the first selected test point for each ear side is presented at its corresponding initial amplitude level, the adaptation offset for adapting the initial amplitude level for the first selected test point for each side and the corresponding offset parameter each are equal to zero.

More preferably, the second predetermined step is the same for all the test points.

Taking the above into account, the offset parameter used for determining the adaptation amplitude offset, for example, for the second selected test point for the left side can be determined as being equal to the offset parameter used for determining the adaptation amplitude offset for the first selected test point for the left side (equal to zero) being increased or decreased by one depending on the response of the subject to the presentation of the first selected test point for the left ear side. If the response is a positive response, the offset parameter used for determining the adaptation amplitude offset for the second selected test point for the left side equals to minus one. If the response is a negative response, the offset parameter used for determining the adaptation amplitude offset for the second selected test point for the left side equals to plus one.

During the evaluation phase, the amplitude level assigned to a selected test point is based on and is also different than the amplitude level assigned to the same test point in its preceding presentation depending on the response of the subject to its preceding presentation.

In particular, it is lower than the amplitude level assigned to the same test point in its preceding presentation, when a positive response is detected and higher than the amplitude level assigned to the same test point in its preceding presentation, when a negative response is detected, preferably by a first predetermined step.

Preferably, the first predetermined step and the second predetermined step are the same. In other words, there is preferably only one predetermined step.

The first predetermined step and/or the second predetermined step is/are advantageously based on data for subjects having the same age and/or gender and/or nationality.

Each test point is being selected/is selectable until a change between the received signals indicating a change between the responses of the subject to two successive presentations of the corresponding test point is detected for the first time (stopping criterion). The hearing threshold for this test point is estimated as the average of the amplitude levels assigned to the test point in said two successive presentations. It is noted that these two successive presentations are also the last two presentations of the test point.

Thus, in the evaluation phase, each selected test point is presented at a different amplitude level than the amplitude level at which the same test point has been presented in its preceding presentation. Each test point is presented to the subject until a change in the responses of the subject to two successive presentations of the same test point is detected for the first time (stopping criterion).

Thus, the present aspect of the invention proposes that the stopping criterion in threshold estimation of each of the targeted test points should be only considered a quest of determining where the boundary of audibility and inaudibility is with the least number of trials and/or with minimal excitation energy being transferred to subject's ear. This is the best way of ensuring that the resulting raw data of an audiometric session that form the audiogram will be biased in the least way.

As mentioned above, in the evaluation phase, each selected test point is presented at a different amplitude level than the amplitude level at which the same test point has been presented in its preceding presentation. If the method comprises only an evaluation phase, the preceding presentation refers to a presentation of the test point in the evaluation phase. That is, all the presentations of a selected test point take place in the evaluation phase. In the first presentation of each test point, the corresponding test point can in particular be presented at the corresponding initial amplitude level. The initial amplitude level can be the same or different for each test point.

If the method comprises both an evaluation phase and an initialization phase, the preceding presentation of a selected test point for the first presentation of the test point in the evaluation phase advantageously refers to a presentation of the test point in the initialization phase.

When the method comprises both an evaluation phase and an initialization phase, the amplitude level assigned to a selected test point for its first presentation during the evaluation phase is based on and is also different than an amplitude level assigned to the test point for its presentation during its initialization phase depending on the response of the subject to its presentation in the initialization phase.

Preferably, the amplitude level assigned to the selected test point for its first presentation during the evaluation phase corresponds to the amplitude level assigned to the test point for its presentation during its initialization phase being increased, in the case of a negative response, or decreased in the case of a positive response of the subject to the presentation of the test point in the initialization phase, by a first predetermined step.

In particular, the amplitude level for the presentation of a selected test point in its first presentation during the evaluation phase is calculated after the presentation of the selected test point in the initialization phase, and in particular after receiving the subject's response to the presentation of the selected test point in the initialization phase.

The proposed methodology according to a preferred embodiment is initialized with amplitude levels and predetermined steps for each test point that are based on data for subjects having the same age and/or gender and/or nationality for each test point or/and with subject's data from previous audiometric sessions. Moreover, the initialization phase provides quickly, a very close estimate of the subject's hearing profile. The following evaluation phase makes use of the aforementioned stopping criterion and together with proper first predetermined steps (for each test point) results in threshold estimation of increased accuracy within a small timeframe. Therefore, a radical increase of the test points being utilized in an audiometric session produces audiograms that better represent the overall auditory health status of the subject, while providing excellent test re-test reliability.

Preferably, the plurality of test points is determined over at least a portion of an audible frequency spectrum by using a distribution according to a musical temperament. This means that the plurality of test points is preferably determined over a portion of an audible frequency spectrum or the complete audible frequency spectrum by using a distribution according to a musical temperament.

It is noted that a musical temperament refers to a tuning system for the subdivision of the octave. Keeping that in mind, the advantage of using a musical temperament for determining the plurality of test points is that the test points are chosen/distributed over the portion of the audible frequency spectrum in a way that makes sense. The expression "makes sense" means/implies the fact that the only relation of human behavior relating frequencies in Hz is found inside the various musical temperament tuning systems. The reason is that in all continents and civilizations, humans have always expressed themselves through music as a hardwired act of their evolution. First music behavioral experimenters such as Pythagoras have revealed specific mathematical relations about how humans sense pitches. In all music theories, an octave is the interval between one musical pitch and another with half or double its frequency. This octave relation between two pitches results in a natural phenomenon that these pitches belong to the same pitch class, as their perceptual imprint feels the same.

It is pointed out that the determination of the plurality of test points by using a musical temperament does advantageously not imply anything on the frequency value of the test point with the lowest frequency and/or on the frequency value of the test point with the highest frequency of the portion of the audible range, over which the test points are distributed. It "only" means that the distribution of the test points between the lowest frequency and the highest frequency over the portion of the audible frequency range, regardless of the values that these frequencies might have, is dictated by a musical temperament.

The audible frequency spectrum more particularly comprises frequencies from 1 Hz to 40 KHz, preferably from 20 Hz to 20 KHz. In this case, the expression "a portion of an audible frequency spectrum" means a portion of the range between 1 Hz and 40 KHz, preferably between 20Hz and 20 KHz.

Preferably, the musical temperament is chosen out of a plurality of musical temperaments based on subject-specific information. The subject-specific information preferably comprises information on the nationality and/or the place of origin and/or place of residence of the subject to be tested. Although an octave is the essential basis in all musical cultures, regional music theories may differ from each other in which and how many intervals an octave is divided into i.e. which scale is used. In other words, different nations may use their own traditional musical tuning systems. This means that a musical temperament may carry "regional information". Thus, even if the "western twelve-equal temperament" is dominating the world, choosing a musical temperament out of a plurality of musical temperaments based on said subject-specific information for distributing the test points over at least a portion of an audible frequency range may be advantageous, as the determination of the test points can be individually adapted to the subject being tested.

Preferably, the musical temperament can be chosen among the Pythagorean tuning, just intonation, the mean-tone temperament and the equal temperament. These are the four principal tuning systems.

According to an embodiment of the present invention, the amplitude level at which a selected test point is presented to the subject during a presentation of the test point is constant during the current presentation of the test point. This particularly means that the amplitude level at which a test point is presented to the subject corresponds to a target level from the beginning of the presentation of the test point until the end of the presentation of the test point to the subject.

According to an alternative preferred embodiment of the invention, the amplitude level assigned to a selected test point for a presentation of the test point varies over/with time during the current presentation of the test point. In other words, the amplitude level at which a selected test point is presented to the subject during a presentation of the test point preferably varies over/with time during the current presentation of the test point. This particularly means that the amplitude level does not correspond to a target level from the beginning of the presentation of the test point until the end of the presentation of the test point to the subject. In particular, the amplitude level preferably starts from a minimum level (first base level) and then reaches a target level. Further, it is preferred that the amplitude level reaches a minimum level (second base level) after the test point has been presented at a target level to the subject.

Preferably, the assigned amplitude level (or in other words the amplitude level at which a selected test point is presented to the subject) is set to reach a target level in two steps. The amplitude level is increased in a first step from a first base level to a first intermediate level and in a second step from the first intermediate level to the target level. It is apparent that the first intermediate level is larger than the first base level and smaller than the target level for a test point. The first intermediate level can also be described as "attack level" within the scope of the present invention. Further, the period for increasing the amplitude level from the first base level to the first predetermined level can also be described as "pre-attack period". Further, the period for increasing the amplitude level from the first predetermined level to the target level can also be described as "attack period". The first base level is advantageously an amplitude level, at which the subject cannot hear the corresponding sound. For example, the first base level can be equal to -50dB.

The two-step increase of the amplitude level from the first base level to the target level is advantageous as the pre-attack period ensures that any amplitude increase from the first base level to the target level will be done in a safe way. Otherwise, in the scenario of one-step attack, a sudden amplitude level increase from the first base level, i.e. the "near silence" level, to a possibly loud presentation stimulus, could either produce audio artifacts due to the limitations of digital-to-analog audio converters that are required to generate the tone or/and could lead to a sudden excitation "overload" to the subject's ear. This acoustically is experienced as an "audio glitch".

Preferably, a duration of a period of the increase of the amplitude level from the intermediate level to the target level and/or an amplitude level increase rate and/or a difference between the intermediate level and the target level during said period are the same, respectively, for the presentation of every test point to the subject. This means that all acoustic stimuli will be presented to the subject's ear within the same timeframe and with the same amplitude increase rate. As a consequence, all acoustic stimuli will transfer excitation energy to the selected tonotopy of the ear in the same manner. In physiology, tonotopy is the spatial arrangement of where sounds of different frequency are processed in the brain

The amplitude level assigned to a selected test point is set to decrease to a second base level in two steps after a presentation of the selected test point to the subject at an amplitude level equal to a target level. Preferably, the amplitude level is set to decrease in a first step from the target level to a second intermediate level and in a second step from the second intermediate level to the second base level. Thus, after a test point is presented to the subject at an amplitude level equal to a target level, the amplitude level is preferably decreased to the second base level in two steps. The amplitude level is preferably decreased in the first step from the target level to the second intermediate level and in the second step from the second intermediate level to the second base level. The second intermediate level can also be described as "release level" within the scope of the present invention.

Similar to the first base level, the second base level is advantageously an amplitude level, at which the subject cannot hear the corresponding sound. For example, the second base level can be equal to - 50dB.

The first base level is advantageously equal to the second base level within the same presentation of a test point. The first intermediate level is preferably different from the second intermediate level within the same presentation of a test point. Advantageously, the first intermediate level is higher than the second intermediate level.

A duration of a period of the increase of the amplitude level from the first intermediate level to the target level is preferably shorter than the duration of the period of the decrease of the amplitude level from the target level to the second intermediate level. This configuration is advantageous because the transition from the first intermediate level, i.e. the attack level, to the target level has been chosen to have a short duration or in other words "attack" time, because, as its name implies, it is perceived as an "attack" and thus ensures the readiness of the subject to be tested. On the contrary, the transition from the target level to the second intermediate level, i.e. the release level, has a longer duration or in other words release time in order to reward subjects for "their positive reaction" by allowing excitation energy to dissipate at a smoother rate.

A duration of a period of the decrease of the amplitude level from the target level to the second intermediate level and/or a duration of a period of the decrease of the amplitude level from the second intermediate level to the second base level is/are preferably dependent from the response of the subject to the presentation of the test point.

In particular, the duration of the period of the decrease of the amplitude level from the target level to the second intermediate level in the case of a positive response is advantageously higher than that in the case of a negative response. Further, the duration of the period of the decrease of the amplitude level from the second intermediate level to the second base level in the case of a positive response is advantageously higher than that in the case of a negative response. This has the advantageous effect that, in the case of a positive response, the auditory nerve of the subject gets enough time to relax from the excitation energy received during the presentation of the previous acoustic stimulus before the next acoustic stimulus is presented to the subject. On the other hand, when the response of the subject is negative, this means that the subject has not perceived the acoustic stimulus and thus his/her auditory nerve will not have been excited. Thus, said durations can be smaller compared to the case of a positive response, what contributes to a shorter duration of the overall hearing test.

It is preferred when two successive presentations of test points to the subject are separated by a pause period in the case of a positive response of the subject to the first presentation of the two successive presentations. It is further preferred when two successive presentations of test points to the subject are separated by a pause period in the case of a negative response of the subject to the first presentation of the two successive presentations.

The pause period in the case of a positive response can be equal to the pause period in the case of a negative response. Thus, a simpler method can be achieved.

The use of a single pause period for both the cases of a positive and a negative response is preferred, when the duration of the period of the decrease of the amplitude level from the target level to the second intermediate level in the case of a positive response is higher than that in the case of a negative response and/or when the duration of the period of the decrease of the amplitude level from the second intermediate level to the second base level in the case of a positive response is higher than that in the case of a negative response. Alternatively, the pause period in the case of a positive response can be higher than the pause period in the case of a negative response.

The pause period in the case of a positive response and/or in the case of a negative response can be the same for each test point.

According to a preferred embodiment of the present invention, the amplitude level assigned to a selected test point is modulated according to a ramping profile. In other words, the relationship between the amplitude level assigned to a selected test point and at which the selected test point is presented to the subject and the time during the presentation of the test point is represented by a ramping profile.

The ramping profile may be such that an increase of the amplitude level from the first base level to the first intermediate level and an increase of the amplitude level from the first intermediate level to the target level are done in a linear or/and exponential manner.

Similarly, the ramping profile may be such that a decrease of the amplitude level from the target level to the second intermediate level and a decrease of the amplitude level from the second intermediate level to the second base level are each done in a linear or/and exponential manner. In other embodiments, the ramping profile can follow any other incremental and/or decremental pattern.

Preferably, an amplitude level increase rate during the pre-attack period is higher than an amplitude level increase ratio during the attack period. The amplitude level increase ratio during the pre-attack period corresponds to the slope of the ramping profile between the first base level and the first intermediate level. The amplitude level increase ratio during the attack period corresponds to the slope of the ramping profile between the first intermediate level and the target level.

A positive response of the subject to the presentation of a test point at an amplitude level, i.e. to the presentation of an acoustic stimulus, is preferably registered as positive only if the response is detected/received within a predetermined time window. The predetermined time window is measured from the end of a predetermined reaction time period starting from the moment that the test point is first presented to the subject at an amplitude level equal to a target level. A positive response of the subject to the presentation of a test point at an amplitude level, i.e. to the presentation of an acoustic stimulus, is registered as falsely positive, if the positive response is detected/received outside the predetermined time window. The predetermined time window is more particularly the time window during which a subject's response in the case he/she hears the sound is (normally) expected after the predetermined reaction time period. The predetermined reaction time advantageously reflects the fact that most normal-hearing humans cannot react to any acoustic stimulus faster than a specific time. This reaction includes in particular the process of detection and/or discrimination task of the acoustic stimulus and the physical movement of the finger of the subject to the response input means, e.g. to a button, in order to provide his/her positive response via the user interface.

In particular, if the positive response of the subject is detected/received before the beginning of said predetermined time window, it can be assumed that the subject either gave his/her response before even the test point is presented to him/her at the target level and/or that his/her reaction time was lower than the reaction time of most of the normal-hearing people, i.e. that he/she was faster than most of the normal-hearing people, and/or that the subject responded too late to the previous acoustic stimulus. As these cases are rather improbable or extremely rare, the positive response detected/received before the beginning of said predetermined time window is preferably registered as a false positive response.

If the positive response of the subject is detected/received after the end of said predetermined time window, the response is registered as a false positive response, as in this case it can be assumed that the subject indicated that he/she has heard the sound, probably because his/her reaction time is much slower than that of the most normal-hearing people. In these cases, registering the positive response as truly positive would not correctly reflect the hearing ability of the subject for this test point.

According to a preferred embodiment of the invention, a response input means, via which a subject can provide his/her response to the presentation of an acoustic stimulus to him/her is preferably configured such that the subject has to exert a force, in particular a pressing force (pressure), on it in order to provide his/her response, in particular his/her positive response. Preferably, the response input means comprises force measuring means for measuring the exerted force. In particular, the response input means may comprise a pressure-sensitive button and/or a pressure-sensitive touch screen.

The data processing system is preferably configured to receive a signal representing a force level of the exerted force on the response input means.

If a late release of the response input means is detected, i.e. if the subject fails to release the response input means within a predetermined time window from the time the response input means is acted on, it is further advantageous that a time interval is added at the end of a pause period after the presentation of a test point to the subject at an amplitude level. The predetermined time window thus ends when said pause period ends. In particular, the predetermined time window corresponds at least to the sum of the duration of the period of the decrease of the amplitude level from the target level to the second base level and the duration of the pause period. The additional time interval thus begins with the end of the pause period. The end of the additional time interval corresponds to the moment that a release of the response input means is detected. By adding said additional time interval, it can be made sure that the subject will have the chance to properly respond to the next acoustic stimulus presented to him/her, even if the subject has not released the response input means for providing his/her response to the presentation of the previous acoustic stimulus within the predetermined time window. The addition of said interval time can also be understood in the framework of the present invention as an extension of the pause period.

Preferably, when a positive response of the subject to the presentation of a test point is providable by exerting a force, in particular a pressing force, on a response input means, which comprises force measuring means for measuring the exerted force, the method comprises receiving a signal representing a force level of the exerted force for providing the positive response to the presentation of the test point. If the force level of the exerted force is equal to or higher than a predetermined level, it can be concluded that the subject feels discomfort, to which the subject reflectively reacts by exerting a higher force than expected. In this case, an additional pause period before a subsequent selection of a test point and thus presentation of an acoustic stimulus to the subject can be inserted. Preferably, the force levels can be recorded in a storage means of a data processing apparatus and be considered in a subsequent hearing test. The force levels can preferably be part of the subject's data from previous audiometric sessions (hearing tests).

A further aspect of the invention lies in a data processing system comprising means configured to perform the previously described method.

Further, the present invention relates to an audiometric system that is configured to carry out the previously described method.

The audiometric system advantageously comprises the previously described data processing system.

The audiometric system may preferably also comprise a sound reproducing means for presenting an acoustic stimulus to a subject and/or a response input means, via which a response of the subject to the presented acoustic stimulus is providable to the audiometric system.

The audiometric system may be formed as an audiometer. An audiometer is a dedicated device configured to test the hearing ability of a subject.

The audiometric system may comprise a server and a client.

The data processing system may correspond to the server or the client.

The response input means can preferably be part of a user interface. The user interface may preferably comprise a data input means and/or a display means.

The response input means is particularly configured to receive a response of the subject to the presentation of an acoustic stimulus to him/her and provide it to the data processing system. In case of a positive response, the response input means preferably provides a signal to the data processing means. The data processing means is configured to detect the positive response based on the corresponding signal. In case of a negative response, the response input means may be configured to not provide any signal to the data processing means. The data processing means is configured to detect the negative response based on the absence of a signal within a predetermined time window.

The response input means can advantageously comprise a button and/or a touch screen, which the subject can press in order to provide his/her response to an acoustic stimulus, i.e. that he/she heard the sound presented to him/her.

The data input means can be used to input data, e.g. subject-related data, to the system and is advantageously connected to the data processing apparatus. The data input means may preferably comprise a keypad and/or a keyboard and/or a touch screen.

The display means is configured to display messages, information, an audiogram or the like, i.e. to visualize data. The display means can preferably comprise a touch screen and/or a (normal) screen/display.

The sound reproducing means may comprise a headphone set and/or a loudspeaker and/or earphones and/or any other sound reproducing means that can present an acoustic stimulus to the subject or in other words reproduce a sound. When the sound reproducing means comprises a headphone set and/or earphones, these are preferably provided with a noise cancellation means.

Preferably, when the sound reproducing means comprises a headphone set, the headphone is of a closed type. Thus, ambient noise can be kept to a minimum, as closed headphones are designed to isolate a person's ears from outside noise. Thus, the hearing test could also be conducted at home, if there is no availability of a specialized soundproof compartment or any other suitable room with no or very low ambient noise.

Further, the present invention refers to a computer program comprising instructions which, when the program is executed by a computer, cause the computer to carry out the already described method.

The present invention further refers to a computer-readable medium having stored thereon the computer program described above.

These and further details, advantages and features of the present invention will be described based on embodiments of the invention and by taking reference to the accompanying figures. Identical or equivalent elements and elements which act identically or equivalently are denoted with the same reference signs. Not in each case of their occurrence a detailed description of the elements and components is repeated.
Figure 1 shows a simplified view of an audiometric system according to a first embodiment of the present invention that is configured to carry out the method of the present invention.
Figure 2 shows a simplified view of part of the audiometric system according to the first embodiment of the present invention.
Figure 3 shows a simplified view of an audiometric system according to a second embodiment of the present invention that is configured to carry out the method of the present invention.
Figures 4 to 20 show different aspects of the method of the present invention.
Figures 21(a) and (b) show an example of a hearing test based on the method of the present invention.

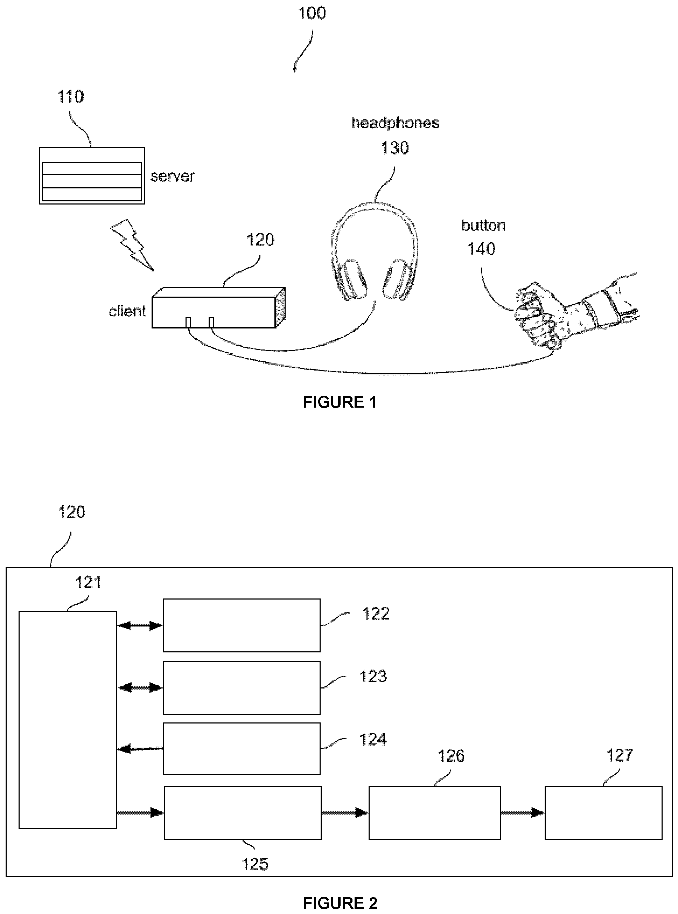

In the following, an audiometric system 100 according to a first embodiment of the present invention is described in detail with reference to figures 1 and 2. The audiometric system 100 is configured to carry out the computer-implemented method according to the present invention for optimizing a hearing test for estimating pure tone hearing thresholds of a subject. The method will be described in detail with references to figures 1 to 21.

Figure 1 is a block diagram illustrating the basic modules of the audiometric system 100 that can be used for conducting a hearing test for estimating the pure tone hearing thresholds of a subject (person).

As can be seen from figure 1, the audiometric system 100 comprises a device 120, a sound reproducing means 130 and a response input means 140. Preferably, the audiometric system 100 further comprises a server 110, wherein the device 120 is formed as a client.

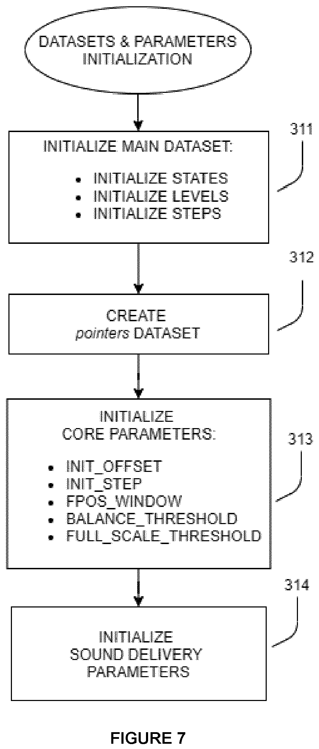

The server 110 is advantageously configured to initialize the device 120. The initialization of the device 120, which will be described in more detail with reference to figure 7, means that initial values for parameters that play a role in the conductance of the method of the present invention can advantageously be derived from the server 110 by the device 120 acting as a client. Thus, it can be ensured that updated initial values, in particular initial values that are specific for the subject to be tested, can be used, what would result in accurate estimations of the hearing thresholds of the subject. However, it is also possible that the initial values can be imported to the device 120 without the need of a server, but directly to the device 120 via a data input means.

The sound reproducing means 130 comprises a headphone set and the response input means 140 a push button. Preferably, the headphone set is an over-ear headphone set, in particular a closed-back headphone set or also called a headphone set of a closed type. This type of headphones helps subjects not get distracted from external "ambient" noises, hence enables subjects to be more concentrated on the test procedure. However, the sound reproducing means 130 may comprise other devices such as open-back headphones, on-ear headphones, in-ear headphones, earbuds, bluetooth headphones, noise-cancelling headphones or bone conduction headphones.

During a hearing test or also known as audiometric session, the subject to be tested interacts with the sound reproducing means 130 and the response input means 140. In particular, the subject wears the headphone set while holding the push button in his/her hand. The audiometric system 100 presents stimuli of various tones (sounds) to the subject's ears via the sound reproducing means 130 and the subject is simply expected to provide his/her response via the response input means, in particular to push the push button, whenever he/she perceives any of the presented tones.

The response input means 140 is preferably equipped with a force sensor that is configured to measure the force level of the force exerted to the response input means 140 by the subject. In the present embodiment, the force sensor is in particular formed as a pressure sensor, which is configured to measure the pressure level applied to the push button used as the response input means 140.

The device 120 of the audiometric system 100 may more particularly be implemented as a dedicated device. The term "dedicated" means that the device 120 is specifically designed to be used for conducting hearing tests, i.e. its purpose is dedicated to the conductance of hearing tests. The device 120 can also be described as an audiometer within the context of the present invention.

Referring now to Figure 2, the device 120 comprises a processor 121, a communication unit 122, a data input means 123, a response input means interface 124, an audio signal generating means 125 or also called digital-to-analog audio converter 125, an amplification unit 126 and a sound output unit 127. The device 120 more particularly corresponds to a data processing system of the audiometric system 100.

The computer program for carrying out the method according to the present invention is implemented within the device 120. However, it is also possible that the computer program is implemented within the server 110. This means in other words that, apart from being used for the initialization of the device 120, the server 110 can be used for carrying out the method according to the present invention. Said computer program can also be called "audiometric screening algorithm" or "auditory nerve evaluation algorithm" within the scope of the present invention.

The communication unit 122 is particularly responsible for the connectivity of the device 120 to the server 110 via the internet. In other words, the communication unit 122 is particularly configured to establish connection and communication between the device 120 and the server 110. Electronically, the communication unit 122 may include all modern connectivity options like Ethernet, Bluetooth, Wi-Fi or cellular modules. If the audiometric system 100 does not include a server, the communication unit 122 can be an optional feature.

The data input means 123 preferably comprises a keypad. The data input means 123 can be used for inputting data to the device 120. The data input means 123 may also comprise a data import port for importing data to the audiometric system 100, in particular the device 120.

The response input means interface 124, which in the present embodiment can also be called button interface, is the communication port to the response input means, in particular the push button, which the subject holds in his/her hand for interacting with the audiometer 100.

The audio signal generating means 125 is a digital-to-analog audio converter and configured to generate acoustic stimuli of different frequencies and amplitude levels. The audio signal generating means 125 is connected to the processor 121 and is configured to receive signals generated by the processor 121 for generating audio signals. In other words, the processor 121 is configured to generate signals to trigger the generation of audio signals by the audio signal generating means 125.

The amplification unit 126 is configured to amplify the audio signals generated by the audio signal generating means 125. The amplification unit 126, which is an optional feature of the audiometer 100, may preferably be used in cases where the full scale line output amplitude level of the chip of the audio signal generating means 125 is still not "loud" enough to be perceived by the subject. This may be the case for subjects, either elderly or with profound hearing loss, whose hearing profile requires amplitude levels that the chip of the audio signal generating means 125 cannot provide without amplification.

The sound output unit 127 is configured to connect the device 120 with and to output the generated audio signals to the sound reproducing means 130. To this end, the sound output unit 127 is connected to the amplification unit 126. If no amplification unit is provided in the device 120, the sound output unit 127 is connected to the audio signal generating means 125. In a modification of the present embodiment of the present invention, the sound output unit 127 may be a part of the communication unit 122 and configured to transmit sound wirelessly.

The sound reproducing means 130 is configured to receive the generated audio signals and produce corresponding acoustic stimuli to be presented to the subject.

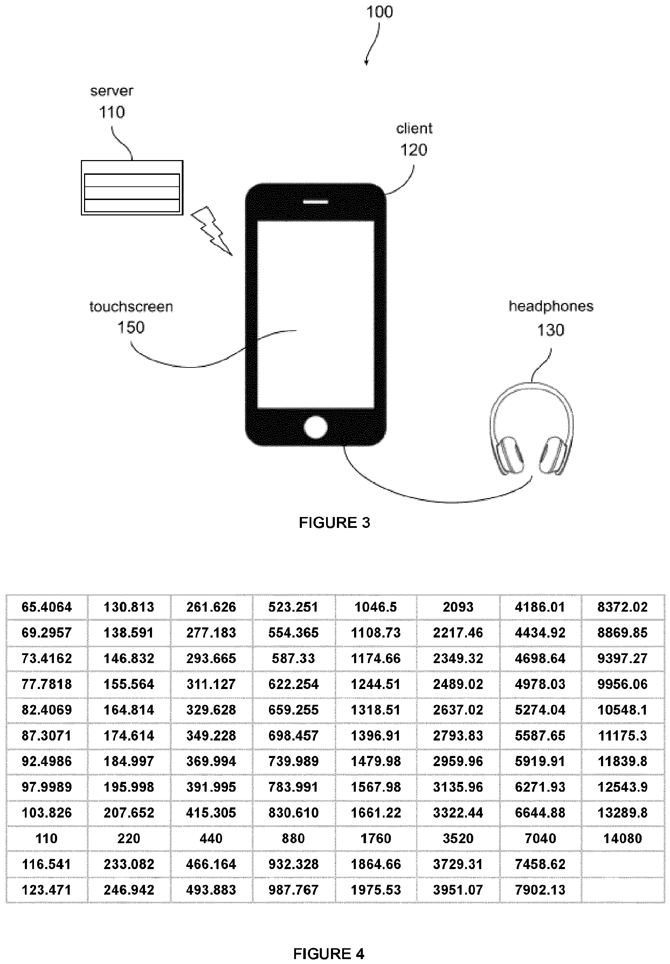

Figure 3 shows an audiometric system 100 according to a second embodiment of the present invention.

Similar to the audiometric system 100 according to the first embodiment, the audiometric system 100 of the second embodiment is configured to carry out the computer-implemented method according to the present invention for optimizing a hearing test for estimating pure tone hearing thresholds of a subject, and comprises a device 120 and a server 110.

However, a main difference between the audiometric system 100 according to the second embodiment and the audiometric system 100 according to the first embodiment is that the audiometric system 100 according to the second embodiment is formed such that the audiometric screening algorithm is implemented in the server 110. In other words, the method of the present invention is performed by the server 110. The server 110 can thus be considered as a data processing system of the audiometric system 100.

The device 120 is formed as a client being configured to communicate with the server 110. In particular, the device 120 is a smartphone comprising a touchscreen 150.

The smartphone corresponding to the device 120 of the audiometric system 100 preferably comprises the modules of the device 120 of the audiometric system 100 according to the first embodiment shown in Figure 2. In particular, the smartphone comprises a processor 121 and a digital-to-analog audio converter 125. The server 110 is configured to generate a signal for triggering the generation of an audio signal by the digital-to-analog audio converter 125 of the smartphone.

The data input means 123 in the smartphone preferably comprises the touchscreen 150 and not a keypad like in the device 120 of the audiometric system 100 of the first embodiment. Subjects may use the touchscreen 150 to input all required data to initialize the audiometric screening algorithm.

The touchscreen 150 may also be used as the response input means 140 of the audiometric system 100. This means that the touchscreen 150 of the smartphone functionally corresponds to the push button of the audiometric system 100 of the first embodiment. Therefore, subjects may use the smartphone's touchscreen 150 to provide their responses to the acoustic stimuli presented to them during the hearing test.

Furthermore, the touchscreen 150 can be used as a display means of the audiometer 100. For example, the touchscreen 150 may be used to display an audiogram of the subject having undertaken a hearing test for having his/her hearing thresholds estimated.

It is further preferred when the touchscreen 150 is pressure sensitive. In this case, the touchscreen 150 may be used to gather pressure data during the press activity of the subject on the touchscreen 150 for providing his/response to the presented acoustic stimuli, as explained later on.

Though the device 120 has been described in the second embodiment of the audiometric system 100 as a smartphone, it is also possible that the device 120 is formed as a personal computer, a pair of hearing aids, a tablet, a smartwatch or another electronic device that incorporates an audio signal generating means 125.

In other embodiments of the present invention, the device 120 could be implemented directly inside a multimedia consumer device like a TV set, a Hi-Fi amplifier, a home theater, a car stereo, etc.

Regardless of its design, the audiometric system 100 of the present invention is configured to determine the subject's hearing threshold at a plurality of test points, which correspond to the sampling points of the audiometric screening session. To this end, the audiometric system 100 is configured to present each test point to the subject at an amplitude level during a presentation of a corresponding test point and to detect the subject's responses to the presentation of the test points.

Every test point targets a specific tonotopy in the subject's ears and is defined by a pair of two parameters: ear side and frequency. In other words, each test point is defined by a frequency value and information about the ear side to be tested. The information about the ear side to be tested indicates which ear side is to be tested in a presentation of a test point to the subject and comprises the options "left ear side" and "right ear side". Every test point can be denoted as t_{s,f}, "s" standing for "ear side" and "f" for frequency.

The presentation of a test point of a specific frequency "f' at a specific amplitude level to an ear "s" of the subject is called an acoustic stimulus. In other words, an acoustic stimulus is the presentation of a test point at a specific amplitude level, called in particular the presentation level. The process of presenting an acoustic stimulus to the subject and detecting the subject's response to the presented acoustic stimulus is called a trial.

During each trial, the audiometric system 100, in particular its corresponding data processing system, determines whether the acoustic stimulus is perceived by the subject or not. In case the subject perceives the acoustic stimulus, it is said that the data processing system detects/receives a positive response. Otherwise, it is said that the data processing system detects/receives a negative response.

The plurality of test points is determined over at least a part of the audible frequency spectrum that the human ear is capable of perceiving by using a distribution according to a musical temperament.

The term "musical temperament" refers to the various tuning systems for the subdivision of the octave. It is pointed out that by distributing the plurality points based on a musical temperament does not necessarily imply anything on the frequency value of the test point with the lowest frequency and/or on the frequency value of the test point with the highest frequency of the portion of the audible range, over which the test points are distributed. It "only" means that the distribution of the test points between the lowest frequency and the highest frequency over the portion of the audible frequency range, regardless of the values that these frequencies might have, is dictated by a musical temperament. In other words, the lowest frequency and the highest frequency of the range of the audible spectrum can be chosen independently from the distribution chosen. However, it might be practical to choose the lowest or highest frequency of the range of the audible spectrum to be tested together with the musical temperament according to which the plurality of test points should be distributed, and choose that the highest or lowest frequency of the range of the audible spectrum, respectively, coincides with a frequency calculated by applying the chosen distribution to the lowest or highest frequency, respectively.

For example, the test points can be distributed by using the twelve-tone equal temperament or also called twelve-tone equal temperament scale. According to this scale, the octave is divided into 12 parts, all of which are equal on a logarithmic scale, with a ratio equal to the 12th root of 2 (≈ 1.05946). The test points can for example be distributed over a frequency range between 65,4064 Hz up to 14080 Hz.

Distributing the test points over said frequency range by applying the twelve-tone equal temperament results in 94 test points per ear side. This means that there are 94 test points for the left ear and 94 test points for the right ear. The specific frequencies of these test points per ear side are shown in Figure 4.

It is understood that other scales such as e.g. the Pythagorean, octatonic, pentatonic, Arabic or Indian scale can be used for the distribution of the test points over a range of the audible spectrum.

Preferably, the musical temperament can be chosen out of a plurality of musical temperaments based on subject-specific information. The subject-specific information preferably comprises information on the nationality and/or the place of origin and/or place of residence of the subject to be tested. The reason behind this is that, apart from world-wide used musical temperaments, each nation may also have its own musical temperaments, and thus, the subject to be tested might be used to the musical temperaments of his/her place of origin and/or place of residence and/or according to his/her nationality.

For the estimation of the hearing thresholds per test point, each test point passes through three successive states: "new", "ready" and "done". A trial for a test point of the plurality of test points can cause the point's transition from one state to another.

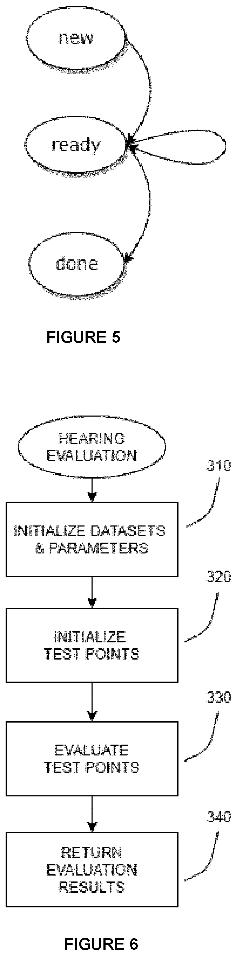

The states "new", "ready" and "done" for each test point and the possible transitions from one state to the other are illustrated in Figure 5.

When a hearing test (audiometric session) is initialized (starts), every test point is in the state "new". When a test point of the plurality of test points is selected and a trial is carried out for the first time for the selected test point, this test point transits from the state "new" to the state "ready".

When a test point is in the state "ready" and the next trial for this test point is carried out, there are two possible outcomes, depending on the response currently detected, when compared to the response detected at the most recent trial for the same test point.

If the response remains the same, the corresponding test point remains at the state "ready". On the other hand, if the response changes from "positive" to "negative" or vice versa, then the corresponding test point transits to the state "done".

When a test point is in the state "done", no more selections and thus presentations, and in particular trials, are carried out for this test point.

The method of the present invention comprises two successive phases, an initialization phase followed by an evaluation phase. During both phases, all test points are selected and amplitude levels are assigned to them. A signal for triggering the generation of an audio signal based on a selected test point and its assigned amplitude level is generated for each selected test point.

The aforementioned transitions from one state to the other for the test points take place during the initialization phase and the evaluation phase. The exact relationship between the initialization and the evaluation phase and the different possible states of the test points is explained in the following.

In the initialization phase, all test points proceed from the "new" state to the "ready" state.

The initialization phase consists of only one trial for each test point. This means that each test point is selected and presented only once to the subject during the initialization phase. In other words, each test point is presented to the subject at only one amplitude level, so that only one acoustic stimulus having a specific frequency per ear side is presented to the subject. The initialization phase is ended when all test points are selected and presented to the subject and the corresponding responses of the subject to the presentation of the test points are detected.

Advantageously, each test point is selected in a random way, so that the subject cannot predict the frequency and the ear side of the next acoustic stimulus that will be presented to him/her. As each test point is selected and presented only once to the subject during the initialization phase, the random selection of the test points is actually a semi-random selection. This means that during the initialization phase a selected test point and in particular the combination of the selected test point and its assigned amplitude level cannot be selected again for being presented to the subject.

It is noted that each test point is associated with a corresponding initial amplitude level. The initial amplitude levels for the test points are advantageously based on data for subjects having the same age and/or gender and/or nationality and/or on data from hearing tests previously conducted for the same subject.

During the initialization phase, the amplitude level assigned to the first selected test point for each ear side is equal to its corresponding initial amplitude level. The amplitude level assigned to any other selected test point of the plurality of test points is equal to an amplitude level, which is based on the initial amplitude level for the corresponding test point and the responses of the subject to the presentation of all test points for the same ear side with that of the corresponding test point that have already been selected in the initialization phase. Thus, in the initialization phase, the first selected test point for each ear side is presented to the subject at its corresponding initial amplitude level. Accordingly, any other selected test point of the plurality of test points is presented to the subject at an amplitude level, which is based on the initial amplitude level for the corresponding test point and the responses of the subject to the presentation of all the already presented test points for the same ear side in the initialization phase. This means that for determining the amplitude level, at which a selected test point other than the first selected test point for each ear side is presented in the initialization phase, the responses of the subject to the presentation of all test points for the same ear side that have been presented so far in the initialization phase are taken into account.

For example, the first selected test point for the left ear is presented to the subject at its corresponding initial amplitude level. The second selected test point for the left ear is presented at an amplitude level, which is based on the initial amplitude level for the second test point and the response of the subject to the presentation of the first test point for the left ear side. The third selected test point for the left ear is presented at an amplitude level, which is based on the initial amplitude level for the third test point and the response of the subject to the presentation of the first and second test points for the left ear side and so on. Accordingly, the first selected test point for the right ear is presented to the subject at its corresponding initial amplitude level. The second selected test point for the right ear is presented at an amplitude level, which is based on the initial amplitude level for the second selected test point and the response of the subject to the presentation of the first selected test point for the right ear side. The third selected test point for the right ear is presented at an amplitude level, which is based on the initial amplitude level for the right selected test point and the response of the subject to the presentation of the first and second selected test points for the right ear side and so on.

In other words, the initial amplitude level of a subsequent selected test point other the first selected test point for each ear side is adjusted before the subsequent selected test point is presented to the subject, by taking into account feedback from every selected test point initialized so far. This procedure runs independently for every ear side.

In particular, in the initialization phase, any selected test point of the plurality of test points is presented at an amplitude level, which is determined based on the initial amplitude level for the corresponding test point and an adaptation amplitude offset for adapting said initial amplitude level. The adaptation amplitude offset for adapting the initial amplitude level of the first selected test point for each ear side is zero and the adaptation amplitude offset for any other selected test point is based on the responses of the subject to the presentation of all already selected and presented test points for the same ear side in the initialization phase.

The adaptation amplitude offset for adapting the corresponding initial amplitude level of a test point other than the first selected test point is determined based on a second predetermined step for a corresponding test point and an offset parameter for the corresponding test point. The offset parameter used for determining the adaptation amplitude offset for the corresponding test point depends on the responses of the subject to the presentation of all the already presented test points for the same ear side in the initialization phase. In other words, as the offset parameter for the corresponding test point depends on the responses of the subject to the presentation of all the already selected and thus presented test points, in particular for the same ear side, the offset parameter for determining the adaptation amplitude offset for the corresponding selected test point can be determined based on the offset parameter for determining the adaptation amplitude offset for the preceding selected test point and the response of the subject to the presentation of the preceding selected test point. In particular, the offset parameter for determining the adaptation amplitude offset for a corresponding selected test point may equal to the offset parameter for determining the adaptation amplitude offset for the preceding selected test point being increased or decreased by one, depending on the response of the subject to the presentation of the preceding selected test point. If the response is a positive response, the offset parameter is decreased. If the response is a negative response, the offset parameter is increased.

The second predetermined step is the same for all test points.

Thus, the amplitude level that is assigned to any subsequent test point other than the first selected test point for each ear side is decreased if the offset parameter for the corresponding test point is negative and increased if the offset parameter for the corresponding test point is positive.

In the evaluation phase, all test points proceed from the "ready" to the "done" state, and so the audiometric session is terminated.

When a test point is in the "ready" state, the audiometric system 100 actually seeks the level at which the subject's perception regarding this test point changes status. One or more selections and thus trials for each test point during the evaluation phase may be needed for this to happen. The selections for each test point are made, similar to the initialization phase, in a random order.

The amplitude level assigned to a selected test point during the evaluation phase is based on and is also different than the amplitude level assigned to the same test point in its preceding presentation depending on the response of the subject to its preceding presentation.

So, every test point is selected and presented to the subject at least two times throughout the whole hearing threshold estimation procedure; the first presentation when transiting from the "new" state to the "ready" state and, at least one more time, in order to transit to the "done" state. It is understood that the first selection and presentation of every test point takes place in the initialization phase, whereas the at least one more selection and presentation of every test point takes place in the evaluation phase.

As already mentioned, during both the initialization and the evaluation phase, the presentation of the test points to the subject does not follow a predefined order. For this purpose, the audiometric system 100 may comprise a module called randomizer. Before every selection of a test point and in particular a trial, the randomizer selects/provides a test point of state "new", when in initialization phase, or of state "ready", when in evaluation phase. The randomizer is described in detail later on.

In figure 6, the method according to the preferred embodiment of the present invention is schematically outlined.

As can be seen, the method comprises four main steps.

In a first main step 310, datasets and parameters used in the method for estimating the hearing thresholds of a subject are initialized. In a second main step 320, the plurality of test points is initialized. This corresponds to the previously described initialization phase. In a third main step 330, the plurality of test points are evaluated. This corresponds to the previously described evaluation phase. In a fourth step, the evaluation results are returned.

As already mentioned, the first main step 310 takes places for creating and initializing datasets and parameters used throughout the audiometric session. All datasets and parameters are preferably stored in a non-volatile memory, in particular provided in the processor 121, so as to be available to all components of the audiometric system 100.

Figure 7 illustrates all datasets and parameters initializations that take place prior to the selection of any of the test points and the actual hearing test, namely the initialization phase and the evaluation phase, in the first main step 310.

A main dataset created in step 311, hereinafter called the "audiometric database", is used to store a plurality of test points. Each of the test points has three attached properties: a) the state ("new", "ready" or "done") of the test point, b) a level, based on which the amplitude level of the acoustic stimulus will be derived, when the next trial for this test point will take place, and c) a step by which the level of the acoustic stimuli will be modified according to the subject's response the next time this point will be tested.

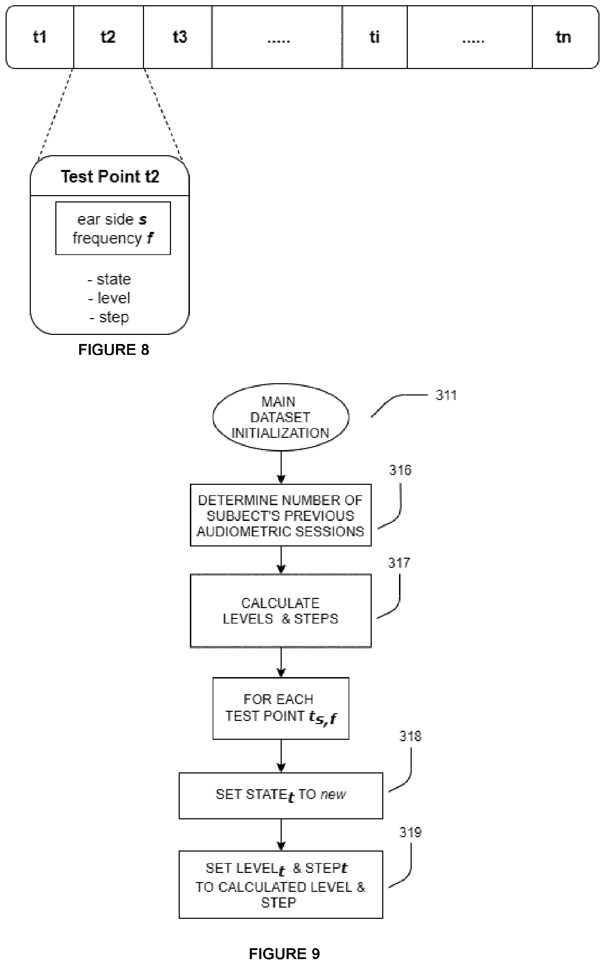

The audiometric database structure in step 311 shown in figure 7 is illustrated in more detail in figure 8.

It comprises n test points, named t1, t2, ..., tn. For illustrative purposes, the second test point is randomly chosen and depicted in detail. As described above, ear side "s" and frequency "f" constitute a test point t_{s,f}, while the three above mentioned properties are attached, namely the "state", the "level" and the "step". It is noted that the value of the property "level" is constantly updated for each test point during the initialization and evaluation phase.

The audiometric database is advantageously initialized with data retrieved from the server 110. However, the audiometric database can also be imported to the audiometric system 100, as previously mentioned.

The value of the property "level" in the beginning of the initialization phase for each test point is equal to the corresponding initial amplitude level for each test point. During the initialization phase, the amplitude level assigned to each selected test point and thus of an acoustic stimulus is derived from the property "level" of the corresponding test point by applying the aforementioned adaptation amplitude offset.

During the evaluation phase, the amplitude level assigned to each test point is derived from the actual value of the property "level" without applying any offset.

After each trial, the properties "state" and "level" of the test point just tested are updated accordingly and are ready for the next trial. The audiometric database initialization process is explained in more detail later on.

In step 312, a pointers dataset is created. It comprises a plurality of pointers, each one referring to a test point. It is being used as a pool, from which the randomizer picks the next test point of the requested state, when asked to. After the trial of this test point has ended and if the test point has changed state, then its pointer is removed from the pool. This means that this test point will not be selected and tested anymore.

Next, five further parameters are advantageously also initialized, as part of step 313: "INIT_OFFSET", "INIT_STEP", "FPOS_WINDOW", "BALANCE_THRESHOLD" and "FULL_SCALE_THRESHOLD".

The first two parameters are "INIT_OFFSET" and "INIT_STEP" and are used only in the initialization phase. "INIT_OFFSET" is a parameter that takes a value, either positive or negative, which is added to the "level" of the test point, before the acoustic stimulus for this point is presented to the subject. The value of the parameter "INIT_OFFSET" in its turn is modified after each trial during only the initialization phase: it is either decreased by "INIT_STEP" if the response is positive or increased by "INIT_STEP" if the response is negative. The modified value of "INIT_OFFSET" is used to adjust the presentation level of the next test point that needs to be initialized. This is happening until every test point has been initialized and has come to the "ready" state.

The modified value of the parameter "INIT_OFFSET" corresponds to the value of the previously described adaptation amplitude offset for each test point. The parameter "INIT_STEP" corresponds to the previously described second predetermined step.

Parameter FPOS_WINDOW is used by the audiometric system 100, when determining the response to an acoustic stimulus. During the audiometric session, the subject may or may not respond to an acoustic stimulus in a timely manner. The subject may even respond to a non-existent acoustic stimulus. The audiometric system 100 filters these responses by setting a specific time window, during which responses are considered valid. The duration of this window is set by parameter FPOS_WINDOW. This process will be further analyzed later on.

The fourth parameter is used by the audiometric system 100 by employing a function in order to provide a balance of the acoustic stimuli presented to the left and the right ear of the subject. Parameter BALANCE_THRESHOLD is used for this purpose and is described in detail later on.

The fifth parameter FULL_SCALE_THRESHOLD is the maximum level that the digital-to-analog audio converter 125 of the audiometric system 100 can provide, and is also explained in detail later on.

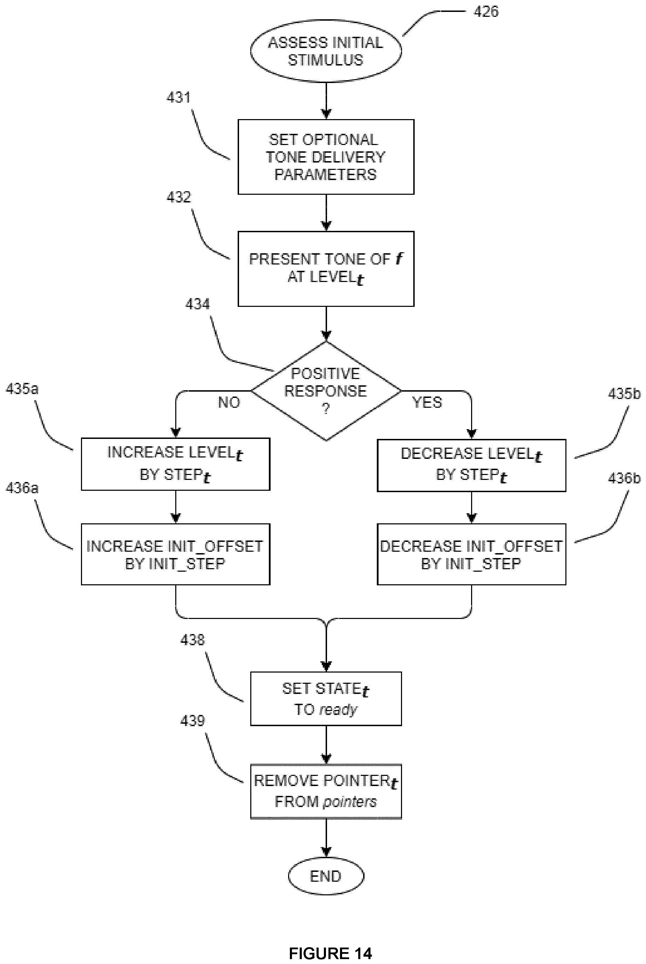

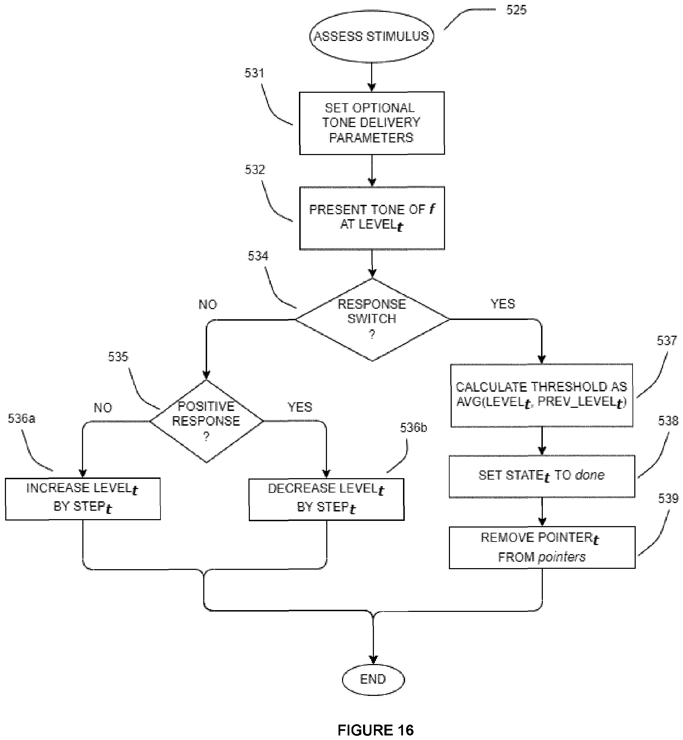

In step 314, parameters that relate to the tonal excitation of the auditory nerve are initialized. These parameters apply in the tone presentation steps 432 and 532 that will be described later on with reference to figures 14 and 16, respectively. Such parameters are "ATTACK_STEP" and "RELEASE_STEP", that relate to amplitude level steps, and "PRE-ATTACK_TIME", "ATTACK_TIME", "TONE_TIME", "RELEASE_TIME", "POST-RELEASE_TIME" or "PAUSE-TIME" that relate to the time durations of the various periods of tonal excitation of the audiometric screening process.

A more detailed illustration of the audiometric database's initialization in step 311 is shown in Figure 9.

In step 316 the audiometric system 100 preferably determines the number of audiometric sessions that have already been completed by the subject. This number is then fed to step 317, where the calculation of the initial amplitude levels and steps take place for each test point. Now, in step 318 the state of each test point is set to "new" and in step 319 the properties "level" and "step" of each test point take their corresponding value from the corresponding elements in the levels and steps dataset, calculated in step 317. Obviously, there should be a match of the frequencies of the subject's audiometric database against the frequencies of the levels and steps dataset.

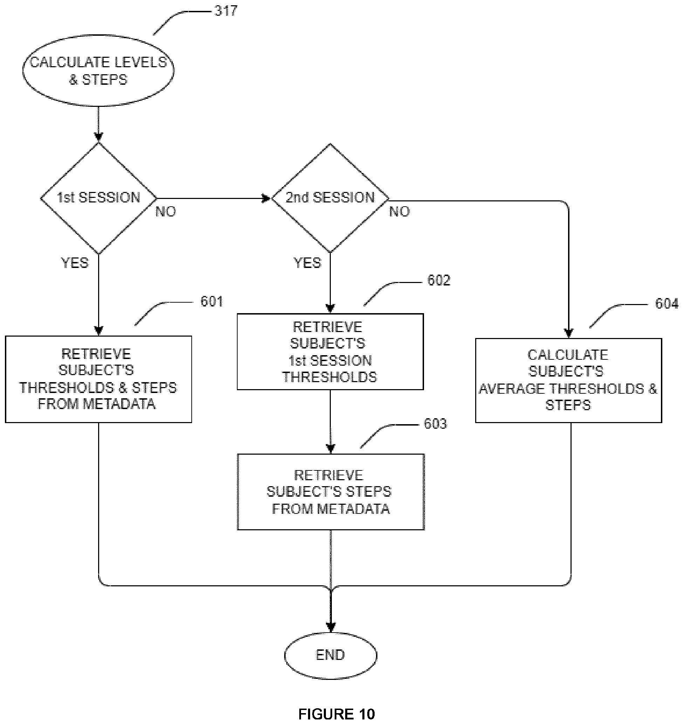

Figure 10 illustrates in more detail the calculations that take place in step 317 for the estimation of initial amplitude levels and steps for the upcoming audiometric session. The audiometric system 100 advantageously uses metadata relating the subject's age, gender and nationality, called audiometric pool.

For the subject's very first audiometric session in step 601, the audiometric system 100 preferably defines the initial amplitude levels and steps from existing data in the audiometric pool, derived from subjects that have the same age, same gender and same nationality with the subject being tested and that were measured in the past. In particular, the initial amplitude level for each of the test points in the first audiometric session of the subject may correspond to an average value of the estimated hearing thresholds for the same test point for subjects with the same aforementioned characteristics with the subject to be tested.

For the subject's second audiometric session in step 602, the estimated hearing thresholds are preferably retrieved from the subject's first audiometric session and used as the initial amplitude levels for the test points, while in step 603, steps are derived from existing data of the audiometric pool derived from subjects having the same age, same gender and same nationality with the subject being tested.

Lastly in step 604, for the subject's third session and for every following audiometric session, the initial amplitude level for each one of the test points is calculated as the average value of all previously estimated hearing thresholds (in the previous audiometric sessions of the same subject) for each specific test point or some of the latest previously estimated hearing thresholds for each specific test point. Consequently, step values of each test point are calculated as the standard deviation of all previous thresholds or some of the latest previous thresholds for each specific test point.

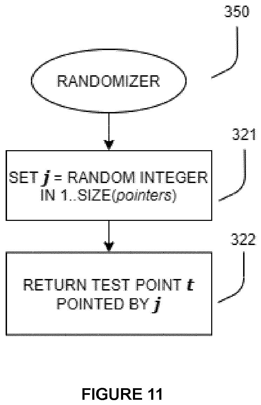

Figure 11 refers to the random selection of each of the test points to be presented to the subject. In particular, the random selection of the test points is carried out by a randomizer 350. The randomizer 350 is implemented within the device 120 or within the server 110.

The randomizer 350 is configured to keep track of every test point's current state. Just before a new trial is started, a random test point of a specific state is requested from the randomizer 350. The requested state takes only two possible values, "new" or "ready", depending on the session phase ("new" in the initialization phase and "ready" in the evaluation phase). The randomizer 350 then returns/selects a new test point *t_{s,t},* but only among the remaining test points of the requested state. At the end of the trial of the selected test point, the state of the test point is properly updated.

In particular, figure 11 illustrates how the above described functions of the randomizer 350 can preferably be implemented.

In step 321, a random integer j is generated, with a value varying between 1 and the size of the pointers dataset. Then, in step 322, the test point t referred by pointer j is retrieved from the audiometric database and returned/selected. At the very beginning of the initialization phase and at the very beginning of the evaluation phase, every test point that the randomizer 350 selects has 50% probability of belonging to the left ear side and 50% probability of belonging to the right ear side. Every other time, it is quite possible that the randomizer will select a "right side" test point with greater probability than a "left side" test point or vice versa.

As explained above, during the evaluation phase, the number of selections and thus trials that are needed for a test point to reach the "done" state is not known a priori. It depends on the subject's hearing profile and its deviation from the initial amplitude levels used.

In case a subject's profile deviates largely from his/her age baseline values, i.e. the expected values for his/her age, this could lead to a phenomenon where the same test point is selected and presented to the subject many times successively or where test points of the same ear side and of adjacent frequencies are selected and presented to the subject one after another. This may be observed only at the first audiometric session and specifically when approaching the end of the hearing evaluation session where the subject could lose the feeling of randomness.

To mitigate this, it is advantageous if a weighing function has been employed by the randomizer 350, which is configured to keep track of the past selections of the test points and thus also trials. If the randomizer 350 detects signs of the case described above, it is configured to "promote" and select test points from the ear side that seems to delay. If, for example, many test points of the left ear side and of adjacent frequencies are selected and presented one after the other, the randomizer 350 will start to select test points of the right ear side.

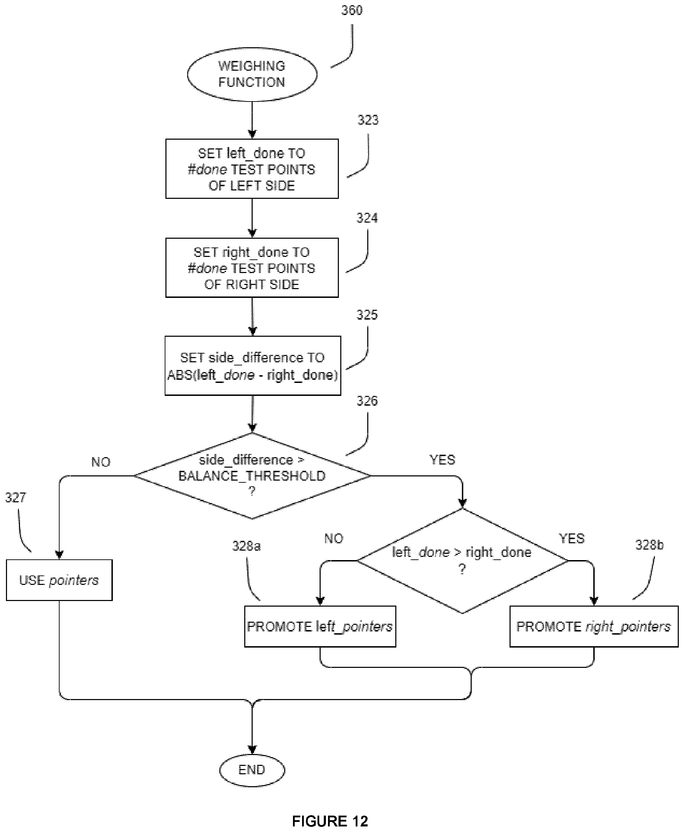

This function is illustrated in detail with reference to Figure 12.

In step 323, the number of test points that have reached the "done" state and belong to the left ear side is counted. In step 324, the number of test points that have reached the "done" state and belong to the right ear side is counted. In step 325, the absolute value of the difference of the two counters previously calculated is found and then compared in step 326 to the parameter "BALANCE_THRESHOLD". This parameter is set in step 313 of the parameters initialization process 310, as previously described.

If this predetermined threshold of "BALANCE_THRESHOLD" is not surpassed, then in step 327 the randomizer 350 uses the pointers dataset and actually functions according to the description in relation to Figure 11. If, on the other hand, the predetermined threshold of "BALANCE_THRESHOLD" is surpassed, the randomizer 350 promotes the ear side with the fewer test points having their state property equal to "done".

To accomplish this, two datasets are introduced, "left_pointers" and "right_pointers", which replace the pointers dataset. These two datasets function exactly like the pointers dataset, apart from the fact that all pointers are now split, depending on the ear side of the test point each pointer refers to. At most times, one dataset is smaller than the other. The smaller dataset is the one that corresponds to the ear side with more test points having their state property equal to "done", while the larger dataset corresponds to the ear side with fewer test points having their state property equal to "done". So, the randomizer 350 picks one of the two datasets by applying a percentage greater than 50% to the larger dataset and a percentage lower than 50% to the smaller one, so as to restore the balance between the test points of the left and right ear side.

A percentage equal to (50+side_difference/2) % is more particularly applied to the larger dataset and a percentage equal to (50-side_difference/2) % is applied to the smaller dataset, where the parameter "side_difference" is the difference calculated in step 325. This difference in the percentages applied to the two datasets is done in steps 328a or 328b, depending on whether the left or the right ear side should be "promoted" respectively. Then, a test point is randomly selected among the test points of the pointers dataset picked above.

It is, however, also possible that a different method is used to calculate the percentages applied to the datasets "left_pointers" and "right_pointers", provided that these sum up to 100%.

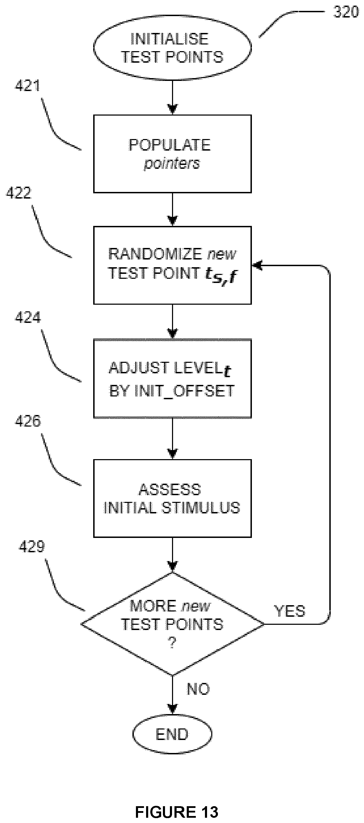

With reference to figure 13, a preferred implementation of the second main step 320 of figure 6, i.e. the test points initialization, is described in detail.

In step 421, the pointers dataset is populated, i.e. a pointer for each test point is added to the pointers dataset.

In step 422, a test point t_{s,f} among all test points having their state property equal to "new" is selected by the randomizer 350.

In step 424, the initial amplitude level for the selected test point that is retrieved from the audiometric database created in step 311 is then adjusted by the value of INIT_OFFSET and stored back to the audiometric database.

Then, in step 426, an audio signal is generated for the selected test point and the adjusted amplitude level that is assigned to the selected test point. The subject is presented with an acoustic stimulus of the adjusted amplitude level and the response of the subject is being assessed. This step will be further analyzed below.

The audiometric system 100 checks in step 429 whether any test points remain having their state property equal to "new". If there are any such test points, a further test point is selected and the above procedure is repeated.

Figure 14 illustrates the initial stimulus assessment of a test point according to step 426. This is the first acoustic stimulus regarding a test point *t_{s,f}* and the process of initial stimulus assessment comprises the following steps.

In step 431, optional tone delivery parameters are being set. These parameters are specific to test point *t_{s,t}.* In step 432, the subject is presented with an acoustic stimulus of frequency *f*, at ear side *s* and at the amplitude level that was calculated in the step 424. For the tone presentation, the audiometric system 100 uses global parameters set in step 314 of the parameters initialization process 310 and any optional parameters set in the previous step 431.

The audiometric system 100 then determines in step 434 whether a positive or a negative response is provided by the subject. In the case of negative response, the level of the test point is increased by the test point's STEP in step 435a. Also, the value of the "INIT_OFFSET" parameter is increased by "INIT_STEP" in step 436a. In the case of a positive response, the level of the test point is decreased by the test point's "STEP" in step 435b and the value of the "INIT_OFFSET" parameter is decreased by "INIT_STEP" in step 436b. The updated value of "INIT_OFFSET" will be used in step 424 of the next test point's initialization trial.

Next in step 438, the test point's state is set to "ready" and in step 439 the pointers dataset is updated, i.e. this point's pointer is removed from the dataset.

The initialization phase, especially for the very first audiometric session, serves as the fastest convergence method towards the hearing profile of all possible subjects, independently of their age or the health status of their auditory nerve. As seen in step 311, and according to the preferred embodiment of the invention, the hearing dataset for the first audiometric session of a subject is initialized with data derived from previously tested subjects having the same age and/or same gender and/or same nationality. This means that it is quite probable that these initial data is close to the hearing profile of the subject being tested. In case this is not true, the above described method can quickly realize this and adapt itself, even from the very first initialization trials. It does not "waste" trials presenting amplitude levels that are probably quite far from the subject's hearing profile and, by means of the "INIT_OFFSET" parameter, this methodology can quickly determine where a subject's audibility and inaudibility boundaries roughly are.

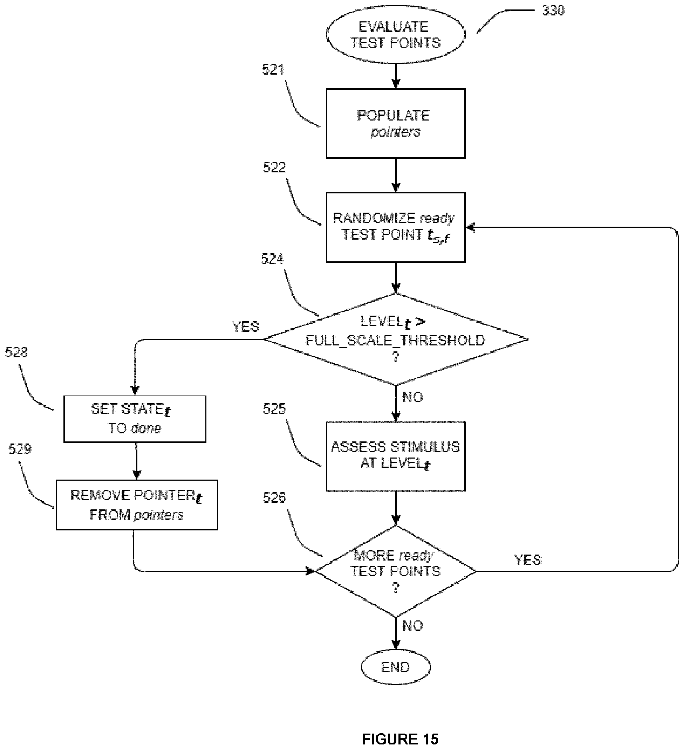

A preferred implementation of the third main step 330 of figure 6 referring to the evaluation phase of the test points is illustrated in Figure 15.

In step 521, the pointers dataset is populated for a second time, i.e. a pointer for each test point is added to the pointers dataset. In step 522, a test point *t_{s,f}* among all test points having their state property equal to "ready" is selected by the randomizer 350. The amplitude level for this test point is retrieved from the audiometric database, and is advantageously checked against the full scale threshold of the digital-to-analog audio converter 125 of the audiometric system 100 in step 524. The full scale threshold level of the digital-to-analog converter 125 is the maximum level that the digital-to-analog converter 125 can provide without distorting its output tonal qualities, hence becoming unable to deliver louder acoustic stimuli.

In case the full scale threshold has not been surpassed, then the subject is presented in step 525 with the corresponding acoustic stimulus and the subject's response is assessed.

In the opposite case, i.e. when the full scale threshold of the digital-to-analog converter 125 has been surpassed, no acoustic stimulus is presented to the subject, this test point's state is set to "done" in step 528 and the pointers dataset is updated in step 529, i.e. this test point's pointer is removed from the dataset. The parameter FULL_SCALE_THRESHOLD indicating the full scale threshold of the digital-to-analog converter 125 is initialized in step 313.

The audiometric system 100 checks in step 526 if there are any test points still having their state property equal to "ready". If there are any such test points, a further test point having a "ready" state is provided and the above procedure is repeated.

In figure 16, a preferred procedure of a stimulus assessment of step 525 is illustrated.

The test point *t_{s,f},* which was provided by the randomizer 350 in step 522 refers to the ear side "*s*" and frequency "*f"* and the process comprises the following steps. In step 531, optional tone delivery parameters are being set. These parameters are specific to test point *t_{s,f}.* In step 532, the subject is presented with an acoustic stimulus of frequency "f", at ear side "s" and at the amplitude level, as retrieved from the audiometric database. For the acoustic stimulus presentation, the audiometric device uses global parameters set in step 314 of the parameters initialization process 310 and parameters set in previous step 531.

The device compares in step 534 the response received by the subject with the response recorded from the most recent trial carried out for test point t_{*s*,*f*}.

In case there was no change, i.e. there were two successive positive or two successive negative responses, two alternative routes exist: If the last response was negative, the amplitude level of the test point is increased by the test point's "STEP" value (first predetermined step) in step 536a. If the last response was positive, the amplitude level of the test point is decreased by the test point's "STEP" value in step 536b.

In case that the two latest responses are different, then the audiometric system 100 determines in step 537 the hearing threshold of the subject for this test point. The state of the test point is set to "done" in step 538 and the pointers dataset is updated accordingly in step 539, i.e. this test point's pointer is removed from the dataset. In the embodiment illustrated in Figure 16, the hearing threshold for this test point is calculated as the average of the amplitude level at which the test point was just tested and the (directly) previous amplitude level, at which the test point was presented, i.e. the amplitude level at which the test point was presented in the directly preceding presentation.

When every test point has reached the "done" state, then the hearing threshold levels of the test points are considered to be estimated (determined). The hearing thresholds are returned in step 340.

As already explained above, during a trial an acoustic stimulus is presented to the subject.

The amplitude level that is assigned to a selected test point and at which the selected test point is presented to the subject during a presentation of the test point varies over/with time during the same presentation of the test point. This particularly means that the amplitude level at which the selected test point is presented to the subject does not correspond to a target (nominal) level from the beginning until the end of the presentation of the selected test point to the subject. In particular, the amplitude level starts from a minimum level (first base level) and then reaches the target level. Further, the amplitude level reaches a minimum level (second base level) after the selected test point has been presented at the target level to the subject. The target level is the maximum amplitude level, at which the selected test point is presented during the corresponding presentation of the selected test point.

In particular, the amplitude level, at which each test point is presented to the subject, i.e. the amplitude level of each of the acoustic stimuli, is modulated according to a ramping profile. This profile is applied to each acoustic stimulus to vary its amplitude over time during the same presentation of the acoustic stimulus.

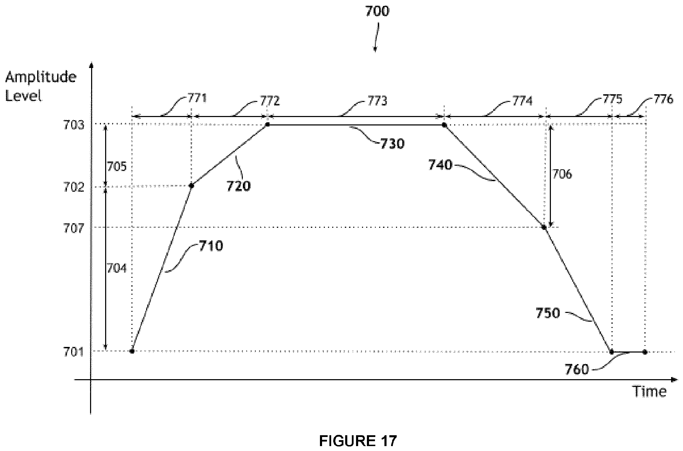

Figure 17 shows the shape of the ramping profile, by which the amplitude level assigned to a corresponding test point and at which the test point is presented, i.e. the amplitude level of each of the acoustic stimuli, is modulated.

As can be seen from figure 17, the presentation 700 of the acoustic stimulus is divided into six periods. The properties that describe each period are its time duration, starting amplitude level, ending amplitude level and the transition function, which describes the way that the amplitude level changes from the starting to the ending amplitude level. The ending point of each period coincides with the starting point of the next period. The amplitude level during a period may be constant or vary in a specific manner.

At any given time, an acoustic stimulus has a value of amplitude, which varies from lower values to higher values and vice versa. During the acoustic stimulus presentation 700, the level of amplitude varies in the range between a base level 701 and a target level 703.

At the beginning of a trial, the acoustic stimulus starts at the base level 701, i.e. at a very low level of amplitude, practically inaudible. The base level 701 can be thought of as a "near-silence" level. It is noted that the amplitude base level 701 cannot be equal to zero, as silence is not the absence of sound. For example, the base level can be equal to - 50dB.

As can further be seen from figure 17, the base level 701 is also the amplitude level reached when the presentation of the acoustic stimulus ends. It is noted that the base level 701 can also be described as a first base level, when referring to the starting amplitude level of the acoustic stimulus, and as a second base level, when referring to the ending amplitude level. It is apparent that, in this case, both the first base level and the second base level correspond to the base level 701. However, it is also possible that the first base level and second base level are different from each other.

The target level 703 actually represents the amplitude level for which the subject is being tested. The target level 703 is determined by the hearing evaluation algorithm just before the presentation of the acoustic stimulus 700 begins.

In figure 17, the different periods of an acoustic stimulus during a stimulus presentation 700 are also shown. These are: pre-attack period 710, attack period 720, tone period 730, release period 740, post-release period 750 and pause period 760.

During the pre-attack period 710, the amplitude level increases from the base level 701 to the attack level (first intermediate level) 702. The pre-attack period 710 has a predetermined but configurable duration 771.

During the attack period 720, the amplitude level increases from the attack level 702 to the target level 703. The attack level 702 is lower than the target level 703 by a value equal to a parameter 705, called "ATTACK_STEP". The attack period 720 has a predetermined but configurable duration 772.

Thus, the amplitude level of the acoustic stimulus being presented to the subject reaches the target level 703 in two steps.

During the tone period 730, the amplitude level remains constant and equal to the target level 703. The period's duration 773 is not known a priori, but a maximum duration is applied. The tone period 773 ends either when the subject responds positively to the acoustic stimulus or when the maximum duration is reached.

During the release period 740, the amplitude level drops from the target level 703 to the release level (second intermediate level) 707 by a value equal to the parameter 706, called "RELEASE_STEP". This period has not a predetermined but a configurable duration 774.

During the post-release period 750, the amplitude level further drops to the base level 701. This period has a predetermined but configurable duration 775.

Thus, after the acoustic stimulus is presented to the subject at the target level 703, the amplitude level is decreased to the base level 701 in two steps.

During the pause period 760, the amplitude level remains constant at the base level 701. The period has a predetermined but configurable duration 776.

During an acoustic stimulus presentation 700, all periods' durations are initialized in the step of the sound delivery parameters initialization 314. The audiometric system 100 uses two attack periods with a duration 772 being considerably longer than a duration 771.

In the first pre-attack period 710, the duration 771 ensures that any amplitude increase from the base level 701 to the attack level 702 will be done in a safe way. Hence, the digital-to-analog audio converter 125 used to generate the acoustic stimuli will be able to handle any possible abrupt amplitude changes. An one-step attack time in the scenario of a sudden amplitude level increase from the base level 701, namely the "near silence" level to a possibly loud acoustic stimulus, could apply a heavy strain in the DSP's capabilities that could lead to a sudden excitation "overload" to the subject's ear. This acoustically is experienced as an "audio glitch".

On the other hand, the second attack period 720 uses the ATTACK_STEP parameter 705. This predetermined constant attack step ensures that all stimuli will be presented to the subject's ear within the same timeframe 772 and with the same amplitude increase rate. As a consequence, all acoustic stimuli will transfer excitation energy from the audiometric system 100 to the selected tonotopy in the subject's ear in the same manner.

The maximum value of the duration 773 mainly depends on the age of the subject. The subjects' reaction time to a perceived acoustic stimulus is largely differentiated upon their age. The proposed audiometric methodology returns normally distributed data regarding an optimal tone duration.

Regarding the durations 774 and 775 illustrated in Figure 17, two parameters for each of them are set: one for the case of a positive response to the acoustic stimulus, when the subject indicates that he/she has perceived the acoustic stimulus and one for the case of a negative response to the acoustic stimulus, when the subject indicates that he/she has not perceived the acoustic stimulus. That is, the time spent for dropping the level from the target level 703 down to the base level 701 depends on whether the subject actually responded positively to the acoustic stimulus or not. These parameters are "RELEASE_TIME_YES" and "POST-RELEASE_TIME_YES", and "RELEASE_TIME_NO" and "POST-RELEASE_TIME_NO", for the two cases respectively. The durations that apply in the negative-response case are smaller than those of the positive-response case, i.e. the amplitude level drops in a faster pace, as explained in more detail below.

In case of a positive response, the audiometric system 100 uses two release periods to drop the amplitude level from the target level 703 down to the base level 701. The first release period 740 uses a constant step, the RELEASE_STEP parameter 706, so that the release of the excitation energy from the subject's ear follows a constant decreasing rate within the RELEASE_TIME_YES timeframe. The second post release period 750 is used to further drop down the amplitude level to the base level 701, as quickly as possible, within the POST-RELEASE_TIME_YES timeframe.

In case of a negative response, the audiometric system 100 also uses two release periods, but this time the RELEASE_TIME_NO duration is set equal to POST-RELEASE_TIME_NO duration so that the hearing test's overall duration is kept to a minimum.

In case of a positive response, PAUSE_TIME_YES duration 776 is set to a non-zero value. If the subject were able to perceive the next acoustic stimulus without a pause time in-between, this would result in two successive positive perceived acoustic stimuli. Hence, an "excitational rest" between these two acoustic stimuli is advantageous. In case of a negative response, it is advantageous to set PAUSE_TIME_NO equal to a non-zero value as well. This is useful mostly in cases where the reason for a negative response is not the subject not having perceived the acoustic stimulus, but an operation of the response input means 140, in particular a pressing of the button 140, happening later than the permitted duration 773.

During an acoustic stimulus presentation 700, the transition function of periods 710, 720 preferably follows an exponential pattern, while the transition function of periods 740 and 750 preferably follow an inverse exponential pattern.

In other embodiments, periods 710, 720 and periods 740, 750 can follow other incremental and decremental patterns, respectively.

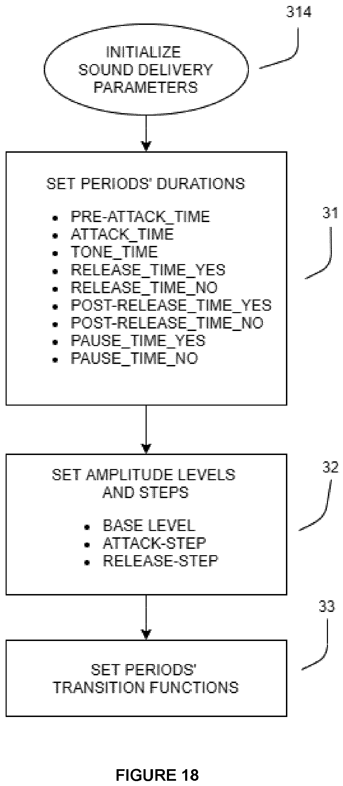

Figure 18 illustrates a detailed view of the initialization of sound delivery parameters of step 314.

As already described, the target level 703 represents the amplitude level, for which each test point t_{s,f} must be assessed. Therefore, its value is derived from the audiometric database in steps 422 and 522 every time the randomizer 350 returns a test point in the initialization and evaluation phase, respectively. The base level 701, ATTACK_STEP 705 and RELEASE_STEP 706 are set in step 314. The attack level 702 is then preferably calculated by subtracting ATTACK_STEP from the target level 703, while the release level 707 is calculated by subtracting RELEASE_STEP from the target level 703.

In particular, the base level 701 may be equal to -50 dB, the ATTACK_STEP 705 equal to 25 dB and the RELEASE_STEP 706 twice as high as ATTACK_STEP, i.e. 50 dB. The transition from the attack level 702 to the target level 703 has been chosen to have a short "attack" duration, because as its name implies it is perceived as an "attack" and thus ensures the readiness of the subjects to be tested. On the contrary, the transition from the target level 703 to the release level 707 preferably has a longer release time to reward the subjects for "their positive reaction" by allowing excitation energy to dissipate at a smoother rate.

It is understood that the base level 701, the parameter ATTACK_STEP 705 and the parameter RELEASE_STEP 706 may have different values than the ones presented above.

Parameters in step 311 are dependent upon the age, gender, nationality and/or other personal information of the subject. These parameters may additionally or alternatively be dependent upon other metadata.

In another embodiment of the invention, sound delivery parameters set at steps 431 or 531 may be used either complementary or to override parameters set at step 314 of the parameters initialization process 310.

Throughout the presentation 700 of the acoustic stimulus, the audiometric system 100 determines the subject's response. If the subject activates the response input means 140, in particular presses the button, during a predefined time window t_{R}, then a positive response is said to be received. If the subject does not activate the response input means 140, in particular does not press the button, then a negative response is said to be received. In both cases though, if the subject activates the response input means 140, in particular presses the button, outside the boundaries of the above predefined time window t_{R}, this is logged as a false positive response.

It is noted that the subject's response is normally expected during the tone period 730. The predefined time window t_{R} is part of this period and only a response that falls into this window is considered valid and is registered.

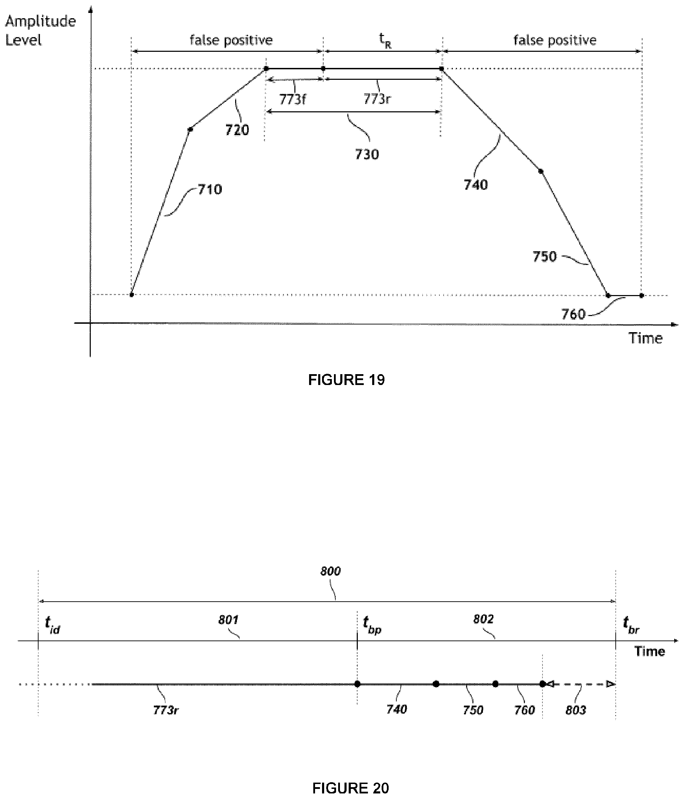

Figure 19 illustrates that the tone period 730 is split into two parts, the second part corresponding to time window t_{R}. The first part has a first duration 773f and the time window t_{R} has a second duration of 773r. The first duration 773f is actually applied to reflect the fact that most normal-hearing humans cannot react to any acoustic stimulus faster than a specific time. This reaction includes the process of detection and/or discrimination task plus the physical movement of the finger of the subject in order to activate the response input means 140, in particular push the button. Thus, an estimate of the subject's overall reaction time can be calculated by adding the time durations of pre-attack period 710, attack period 720 and of the first part of the tone period 773f. The proposed audiometric methodology returns normally distributed data regarding the duration 773f.

The value of duration 773f resides in parameter FPOS_WINDOW, which in the preferred embodiment of the invention is set at step 313 of the parameters initialization process 310.

So, a response of the subject is considered positive only if it is logged within t_{R}. On the other hand, if a response is logged during either the pre-attack period 710, the attack period 720 or the first part of the tone period 773f, it is considered false positive and is not registered as a positive response. Similarly, if a response is logged after the tone period 730 has ended, i.e. during the release, post_release or pause periods, it is also considered a false positive one.

False positive responses logged after time window t_{R} usually refer to cases of the subject's late response to the current acoustic stimulus i.e. approaching the end of the current trial, while false positive responses logged before time window t_{R} usually refer to cases of a very late response to the previous stimulus, i.e at the beginning of the next trial. In both cases, i.e. whenever the subject presses the button 140 outside the time window 773r, this reaction is logged as a false positive reaction.

As illustrated in Figure 20, the button press activity 800 starts at the instant of time t_{id} where the discrimination and/or identification of a stimulus actually takes place. The second instant of time t_{bp} is the moment the subject is pressing down the button. The third instant of time t_{br} is the moment the subject is releasing the button.

As explained above, button press activity is composed of two discrete time periods 801 and 802. These periods together make up reaction time 800, i.e. the time duration it takes subjects to perform a button press as a reaction to a perceived acoustic stimulus.

Discrimination and/or identification time instant t_{id} may occur before window t_{R} starts or after it starts, but what is important is that for the logging of a positive response, the instant of button press t_{bp} should always occur within the permitted time window t_{R}, whose duration is 773r. Although duration 773r is primarily age dependent and is initialized at step 313, reaction time involves as well the release of the button at instant t_{br}, i.e. period 802, which cannot be known a priori. There are cases where subjects keep the button pressed and release it after the stimulus presentation has ended. If that is the case, duration 802 is greater than durations of release 740, post-release 750 and pause 760 periods, summed together. These subjects are usually elderly people and the above case is an indication that they cannot cope with the device's tempo, i.e. cannot follow the device's rate of trials' presentation.

When a subject fails to release the push button in a timely manner and releases it after the next trial has started, then he/she may lose the chance of correctly responding to the next trial. As a consequence, a negative response may be logged for the next trial. This also results in one more acoustic stimulus for the same test point, but of higher presentation level, which will excite even more tonotopies in the subject's ears.

This phenomenon of "psychophysical underbuffer" could bias threshold estimation especially for elderly people. To prevent this from happening and thus further decrease the potential bias in the response of a subject, an additional time interval 803 is simply added to the duration of the pause period 760 of the current trial. This additional pause time interval 803 is initialized whenever a trial ends but the subject has not released the response input means yet and ends the moment the subject finally releases it.

It is noted that subjects may not apply the same amount of pressure during the press activity of the push button for all perceived acoustic stimuli. The reason is that there are usually acoustic stimuli that correspond to tonotopies in the subject's ears, where subjects are most sensitive.. If that is the case, apart from the "acoustic" properties of the stimulus, subjects may also experience a minor discomfort.

As already described with reference to the first embodiment of the audiometric system 100, the push button that subjects use to provide their response to the presentation of a selected test point may preferably be equipped with a pressure sensor, so that the pressure level applied on the button is measured and logged after each response. In the second embodiment of the audiometric system 100, pressure data may be logged if the touchscreen 150 is pressure sensitive. This could give an in-depth view of how the experienced minor discomfort relates to specific tonotopies and their threshold properties.

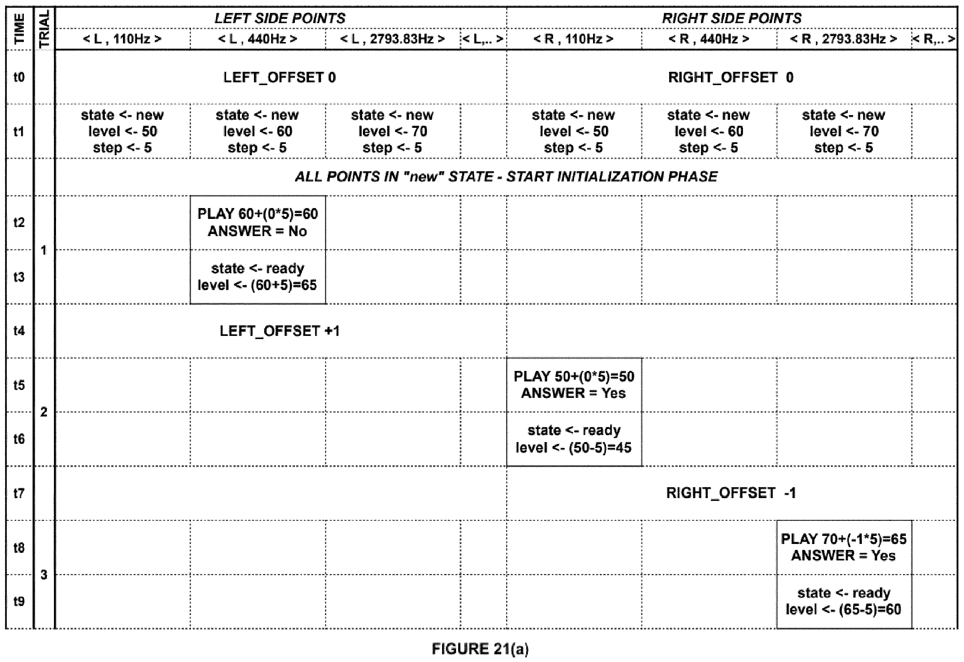

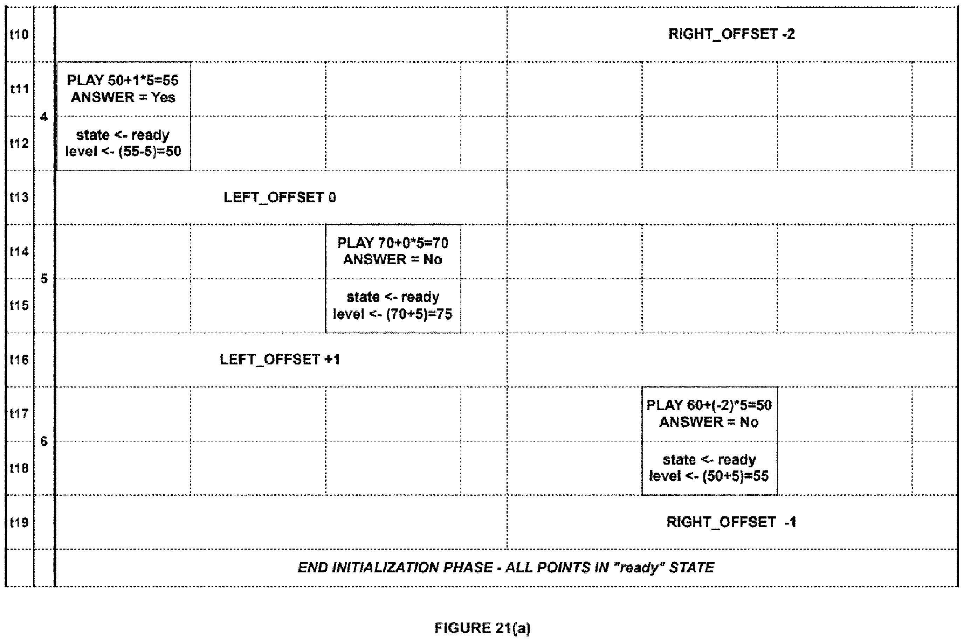

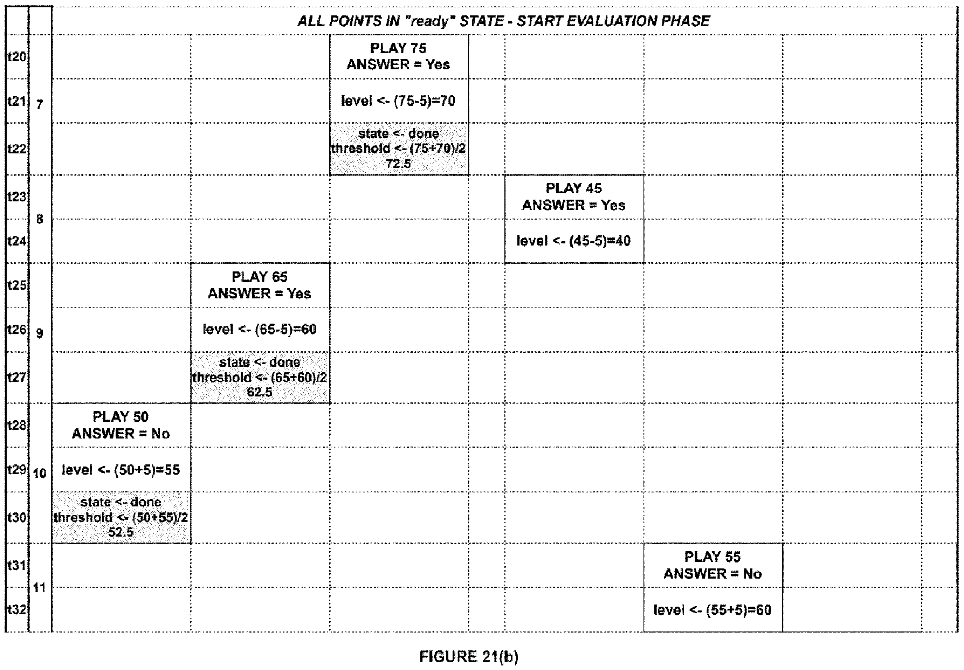

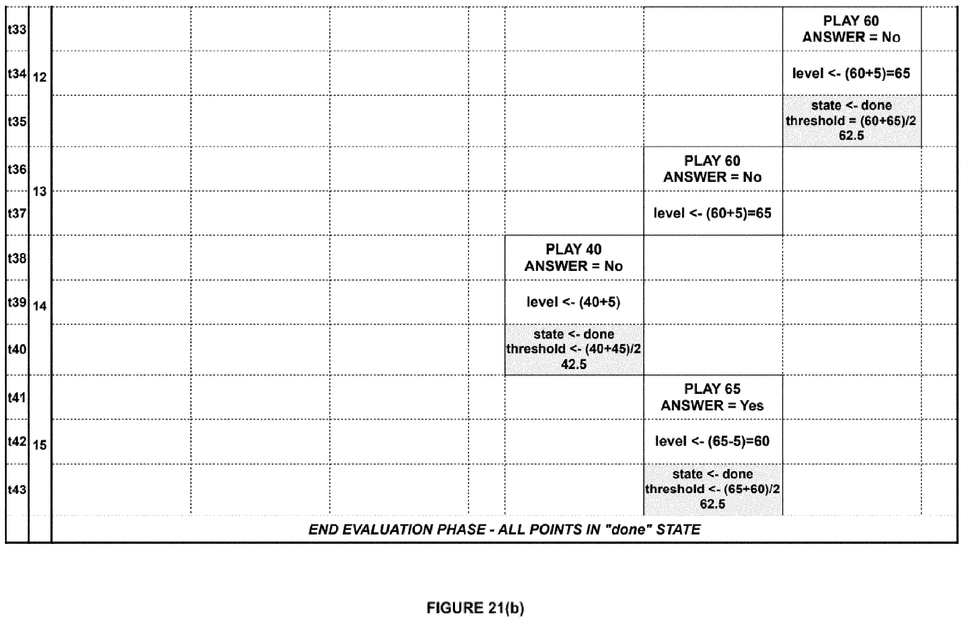

An example of the computer-implemented method for optimizing a hearing test for estimating pure tone hearing thresholds of a subject and of the process of a hearing test according to the present invention is described for a better understanding with reference to the table of figures 21(a) and (b).

The column named "TIME" refers to points in time, at which specific steps of the method take place. The column named "TRIAL" refers to the identification number for each of the trials. It is reminded that a trial includes the presentation of an acoustic stimulus to the subject, i.e. the presentation of a test point at an amplitude to the subject, and a detection of the subject's response to said presentation. It is noted that a trial is presented to also include the adaptation steps of the property "state" of each test point and of the property "level" corresponding to the amplitude level. However, said adaptation steps can also be considered not to belong to a trial. The column named "LEFT SIDE POINTS" with it subordinated columns refers to the test points for the left ear, while the column named "RIGHT SIDE POINTS" with its subordinated columns refers to the test points for the right ear. For example, the notation "L, 110 Hz" means that the test point is defined by a frequency of 110 Hz and the left ear side. Accordingly, the notation "R, 110 Hz" means that the corresponding test point is defined by a frequency of 110 Hz and the right ear side. It is noted that only some of the test points are included in the table, as the table is only intended to outline the method based on an example.

Both the initialization phase and the evaluation phase are presented in the table. The initialization phase is presented in figure 21(a) and the evaluation phase in figure 21(b).

After each trial in the initialization phase, the adaptation of the parameters "LEFT_OFFSET" and "RIGHT_OFFSET" is presented.

In the following, the test point for the left ear side having a frequency of 110 Hz, the test point for the left ear side having a frequency of 440 Hz and the test point for the left ear side having a frequency of 2793,83 Hz are indicated as the first left test point, second left test point and third left test point, respectively. Accordingly, the test point for the right ear side having a frequency of 110 Hz, the test point for the right ear side having a frequency of 440 Hz and the test point for the right ear side having a frequency of 2793,83 Hz are indicated as the first right test point, second right test point and third right test point, respectively. It is pointed out that the indication of the test points as "first left/right", "second left/right" and "third left/right" refers to the appearance of the test points in the table and not to their order of presentation to the subject, as will also be made apparent later.

At t0, the parameters "LEFT_OFFSET" and "RIGHT_OFFSET" are initialized. These parameters refer to the offset parameters previously described for the left ear side and the right ear side, respectively. Both parameters have the value "zero" at the beginning of the hearing test.

At t1, the properties "state", "level" and "step" for each test point are initialized.

Initially, all test points have the "new" state.

The initial value of the property "level" for each test point is equal to the corresponding initial amplitude level. The initial amplitude levels are based on data from subjects having the same age and/or the same gender and/or the same nationality and/or on data from the subject's previous hearing tests, if available, as has already been described. In the present example, the initial amplitude levels for the first left test point, the second left test point and the third left test point are 50 dB, 60 dB and 70 dB, respectively. Further, the initial amplitude levels for the first right test point, the second right test point and the third right test point are 50 dB, 60 dB and 70 dB, respectively. In general, the value of the property "level" for each test point is updated throughout the initialization phase and evaluation phase and corresponds to the amplitude level assigned to the corresponding test point, at which the test point will be presented to the subject.

The value of the property "step" is equal to the first predetermined step being equal to the second predetermined step and remains constant throughout the whole procedure. In the present example, the value of the property "step" is equal to 5 dB. It is noted that, though in the present example the first predetermined step and the second predetermined step are equal to each other, the second predetermined step is used in the calculation of the amplitude levels assigned to the test points for their presentation in the initialization phase, while the first predetermined step is used in the calculation of the amplitude levels assigned to the test points for their presentations in the evaluation phase. Thus, in the following, the terms "first predetermined step" and "second predetermined step" may be used accordingly.

After the initialization of the aforementioned parameters and properties, the initialization phase starts. It is reminded that during the initialization phase each test point is selected and presented only once to the subject and that the selection of the test points to be presented to the subject is made in a random way.

For example, due to the random selection of the test points to be presented to the subject, the first test point presented to the subject at t2 is the aforementioned second left test point. As this is the first trial for the left ear side and the corresponding offset parameter "LEFT_OFFSET" is zero, the amplitude level assigned to the second left test point corresponds to its initial amplitude level of 60 dB and thus, the second left test point is presented at said initial amplitude level of 60 dB. As can be seen, no response has been received to the presentation of the second left test point to the subject.

At t3, the state of the second left test point is then changed from "new" to "ready", as this test point will be not tested again during the initialization phase. Further, as no response has been detected, the parameter "level" is increased by the first predetermined step. The resulting amplitude level will be the amplitude level, at which the second left test point will be presented in its first presentation during the evaluation phase.

At t4, the value of the offset parameter "LEFT_OFFSET" is increased from zero to one, as there was no response to the presentation of the lastly selected and presented test point for the left ear side, i.e. the second left test point. This value will be used together with the second predetermined step for calculating the amplitude level at which the next selected test point for the left ear side will be presented during the initialization phase. In this case, the next test point that is selected and presented to the subject during trial 4 is the first left test point.

At t5, the second test point that is randomly selected to be presented to the subject is the first right test point. As this is the first trial for the right ear side and the corresponding offset parameter "RIGHT_OFFSET" is zero, the amplitude level assigned to the first right test point corresponds to its initial amplitude level of 50 dB and thus, the first right test point is presented at said initial amplitude level of 50 dB. As can be seen, a positive response has been received to the presentation of the first right test point to the subject.

At t6, the state of the first right test point is then changed from "new" to "ready", as this test point will be not tested again during the initialization phase. Further, as a positive response has been received, the parameter "level" is decreased by the first predetermined step. The resulting amplitude level is the amplitude level, at which the first right test point will be presented in its first presentation during the evaluation phase.

At t7, the value of the parameter "RIGHT_OFFSET" is decreased from zero to minus one, as there was a positive response to the presentation of the lastly presented test point for the right ear side, i.e. the first right test point. This value will be used together with the second predetermined step for calculating the amplitude level at which the next selected test point for the right ear side will be presented during the initialization phase. In this case, the next test point that is selected and presented to the subject during trial 3 is the third right test point.

This procedure continues for the rest of the test points.

The parameters "RIGHT_OFFSET" and "LEFT_OFFSET" are constantly changing during the initialization phase depending on the response of the subject to the previously presented test point for the right ear side and the left ear side, respectively. As the adaptation of these parameters takes place every time a test point for a corresponding ear side has been presented to the subject, it can be said that the parameters "RIGHT_OFFSET" and "LEFT_OFFSET" used for each test point other than the first selected and presented test point for each ear side are based on the results of all the previously presented test points for the same side.

The initialization phase ends when all test points have been selected and presented once to the subject. At the end of the initialization phase, the amplitude levels at which the test points will be presented during their first presentation in the evaluation phase have been calculated and all the test points are in the "ready" state.

After the end of the initialization phase, the evaluation phase for estimating the hearing thresholds of the subject for each test point begins.

During the evaluation phase, each test point out of the plurality of test points to be presented to the subject is selected, similar to the initialization phase, in a random way.

The amplitude level assigned to a selected test point for its first presentation during the evaluation phase corresponds to the calculated amplitude level after the presentation of the same test point in the initialization phase as explained above. In particular, said amplitude level corresponds to an amplitude level resulting by increasing or decreasing the amplitude level at which the corresponding test point has been presented in the initialization phase by the first predetermined step, if the response of the subject to the presentation of the test point in the initialization phase was negative or positive, respectively. The amplitude level that is assigned to a selected test point for any presentation of the test point other than its first presentation in the evaluation phase is higher or lower than the amplitude level, at which the same test point has been presented in its preceding presentation in the evaluation phase, by the first predetermined step, if the response to the preceding presentation of the test point in the evaluation phase was negative or positive, respectively.

As can be seen from figure 21(b), the third left test point has randomly been selected to be presented first to the subject in the evaluation phase at t20. The amplitude level of 75 dB that is assigned to the third left test point and at which the third left test point is presented to the subject has previously been calculated in the initialization phase according to the procedure described above.

A positive response of the subject is received/detected for the presentation of the third left test point at said amplitude level. Due to this, at t21, the current value of the property "level" for the third left test point is decreased by the first predetermined step. The resulting amplitude level would be the amplitude level that would be assigned to the test point for its next presentation.

However, as there was a change detected in the response of the subject to the presentation of the third left test point between its two last successive presentations (negative response in the initialization phase and positive response in the evaluation phase) and this change is detected for the first time for said test point, the state of the test point is changed from "ready" to "done" at t22. Thus, no further selections and consequently presentations take place for the test point. The hearing threshold is determined as the average of the amplitude levels, at which the test point was presented to the subject in these two successive presentations, which also are the two last presentations of the test point. As the test point was presented to the subject at 70 dB in the initialization phase and at 75 dB in the evaluation phase, the hearing threshold for this test point is defined as 72,5 dB. It becomes apparent that the hearing threshold for the third left test point has been estimated after two presentations of the test point to the subject, the first one having taken place in the initialization phase and the second one in the evaluation phase.

The next test point that is randomly selected to be presented to the subject is the first right test point.

At t23, said test point is presented to the subject at an amplitude level of 45 dB, calculated during the initialization phase, and a positive response of the subject to this presentation is detected. Due to the positive response, the amplitude level, at which the test point will be presented to the subject during its next presentation, is calculated by decreasing the current value of the parameter "level" by the first predetermined step. Thus, the resulting amplitude level equals to 40 dB. It becomes apparent at this stage that more than two presentations are needed for the estimation of the hearing threshold for the first right test point, as the responses of the subject to the presentation of the first right test point during the initialization phase and the evaluation phase were both positive. As no change in the responses of the subject to the presentation of the first right test point between the aforementioned presentations is detected, the first right test point can and will be selected again during the evaluation phase in order to estimate the hearing threshold for the first right test point.

The next test point that is randomly selected to be presented to the subject is the second left test point.

At t25, said test point is presented to the subject at an amplitude level of 65 dB, calculated during the initialization phase, and a positive response is detected. Due to the positive response, the current value of the property "level" is decreased by the first predetermined step. However, as there was negative response to the presentation of the second left test point during the initialization phase, whereas there is a positive response to the current presentation, a change in two successive presentations of the same test point is detected for the first time, and thus, the state of the test point is changed from "ready" to "done" at t27. This means that no further selections and consequently presentations for said test point will be made and that the hearing threshold for this test point is equal to 62,5 dB. This corresponds to the average of the amplitude levels of these two successive presentations, which are also the two last presentations of the test point, namely 60 dB during the initialization phase and 65 dB during the evaluation phase. Similar to the third left test point, the hearing threshold for the second left test point has been estimated in the present example after two presentations of the test point to the subject, the first one having taken place in the initialization phase and the second one in the evaluation phase.

It becomes apparent from figures 21(a) and (b) that the hearing threshold for the first right test point is estimated in trial 14 after two selections and presentations of said test point in the evaluation phase and after five other test points have been selected and presented to the subject in between (the second left test point in trial 9, the first left test point in trial 10, the second right test point in trial 11, the third right test point in trial 12 and again the second right test point in trial13). Thus, in the evaluation phase, when a test point is randomly selected to be presented to the subject, any further selections and presentations of the test point needed for the estimation of the hearing threshold for this test point do not follow a predetermined order but are randomly chosen. In particular, any further selections and presentations of the test point needed for the estimation of the hearing threshold for this test point do not follow the predetermined order, according to which the test point would continuously be presented at different amplitude levels as many times as needed until a change in the responses of the subject to two successive presentations of the test point is detected for the first time.

In addition to the foregoing description of the present invention, for an additional disclosure explicit reference is taken to graphic representation of figures 1 to 21.

## Claims

1. A computer-implemented method for optimizing a hearing test for estimating pure tone hearing thresholds of a subject, comprising the steps of:
• determining a plurality of test points (t_{s,f}), wherein each test point (t_{s,f}) is defined by a frequency value and information about the ear side to be tested,
• selecting a test point (t_{s,f}) of the plurality of test points (t_{s,f}) in a random way,
• assigning an amplitude level to the selected test point (t_{s,f}),
• generating a signal for triggering the generation of an audio signal based on the selected test point (t_{s,f}) and assigned amplitude level for representing a corresponding acoustic stimulus, and
• detecting a response of the subject to the acoustic stimulus to the subject.

2. The method according to claim 1, wherein the combination of the selected test point (t_{s,f}) and the assigned amplitude level is chosen in a random order, in particular a semi-random order.

3. The computer-implemented method according to any of the preceding claims,
• comprising an initialization phase, during which each test point (t_{s,f}) is selected only once,
wherein preferably each test point (t_{s,f}) is associated with a corresponding initial amplitude level,
wherein the amplitude level assigned to the first selected test point (t_{s,f}) for each ear side is equal to its corresponding initial amplitude level, and the amplitude level for any other selected test point (t_{s,f}) of the plurality of test points (t_{s,f}) is equal to an amplitude level, which is based on the initial amplitude level for the corresponding test point (t_{s,f}) and the responses of the subject to the presentation of all test points (t_{s,f}), in particular for the same ear side with that of the corresponding test point (t_{s,f}), that have already been selected in the initialization phase, and/or
wherein the initial amplitude levels for the test points (t_{s,f}) for estimating the hearing thresholds of a subject are based on data for subjects having the same age and/or gender and/or nationality and/or data of previous hearing tests of the subject,
and/or
• comprising an evaluation phase, during which the amplitude level assigned to a selected test point (t_{s,f}) is based on and is also different than the amplitude level assigned to the same test point (t_{s,f}) in its preceding presentation depending on the response of the subject to its preceding presentation, in particular lower than the amplitude level assigned to the same test point (t_{s,f}) in its preceding presentation, when a positive response is detected and higher than the amplitude level assigned to the same test point (t_{s,f}) in its preceding presentation, when a negative response is detected,
and wherein each test point (t_{s,f}) is being selected until a change between the received signals indicating a change between the responses of the subject to two successive presentations of the corresponding test point (t_{s,f}) is detected for the first time, wherein the hearing threshold for this test point is estimated as the average of the amplitude levels assigned to the test point in said two successive presentations,
wherein the method preferably comprises both the initialization phase and the evaluation phase, wherein the amplitude level assigned to a selected test point (t_{s,f}) for its first presentation during the evaluation phase is based on and is also different than an amplitude level assigned to the test point (t_{s,f}) for its presentation during its initialization phase depending on the response of the subject to its presentation in the initialization phase, wherein preferably the amplitude level assigned to the selected test point (t_{s,f}) for its first presentation during the evaluation phase corresponds to the assigned amplitude level assigned to the test point (t_{s,f}) for its presentation during its initialization phase being increased in the case of a negative response or decreased in the case of a positive response of the subject to the presentation of the test point in the initialization phase, by a first predetermined step.

4. The computer-implemented method according to any of the preceding claims, wherein the plurality of test points (t_{s,f}) is determined over at least a portion of an audible frequency spectrum by using a distribution according to a musical temperament,
in particular:
wherein the musical temperament is chosen out of a plurality of musical temperaments based on subject-specific information, wherein the subject-specific information comprises information on the nationality and/or the place of origin and/or place of residence of the subject to be tested, and/or
wherein the musical temperament is one of the Pythagorean tuning, just intonation, the mean-tone temperament and the equal temperament.

5. The computer-implemented method according to any of the preceding claims, wherein the amplitude level assigned to a selected test point (t_{s,f}) for a presentation of the test point (t_{s,f}) is set to vary over time during the same presentation of the test point (t_{s,f}), wherein preferably the amplitude level assigned to a selected test point (t_{s,f}) is modulated according to a ramping profile.

6. The computer-implemented method according to claim 5, wherein the assigned amplitude level is set to reach a target level (703) in two steps, wherein in a first step the amplitude level is set to increase from a first base level (701) to a first intermediate level (702) and in a second step from the first intermediate level (702) to the target level (703),
in particular wherein an excitation energy transferred to the subject during the increase of the amplitude level from the first intermediate level (702) to the target level (703) is delivered in the same timeframe for the presentation of every test point (t_{s,f}) to the subject and/or wherein preferably a duration of a period (772) of the increase of the amplitude level from the first intermediate level (702) to the target level (703) and an amplitude level increase rate during said period are the same, respectively, for the presentation of every test point (t_{s,f}) to the subject.

7. The computer-implemented method according to claim 5 or 6, wherein the amplitude level assigned to a selected test point (t_{s,f}) is set to decrease to a second base level (701) in two steps after a presentation of the selected test point (t_{s,f}) to the subject at an amplitude level equal to a target level (703), wherein in a first step the amplitude level is set to decrease from the target level (703) to a second intermediate level (707) and in a second step from the second intermediate level (707) to the second base level (701).

8. The computer-implemented method according to claim 7, wherein a duration (774) of a period of the decrease of the amplitude level from the target level (703) to the second intermediate level (707) and/or a duration (775) of a period of the decrease of the amplitude level from the second intermediate level (707) to the second base level (701) is/are dependent from the response of the subject to the presentation of the test point (t_{s,f}), in particular wherein:
the duration (774) of the period of the decrease of the amplitude level from the target level (703) to the second intermediate level (707) in the case of detecting a positive response is higher than that in the case of detecting a negative response
and/or
the duration (775) of the period of the decrease of the amplitude level from the second intermediate level (707) to the second base level (701) in the case of detecting a positive response is higher than that in the case of detecting a negative response.

9. The computer-implemented method according to any of the preceding claims, wherein two successive presentations of test points (t_{s,f}) to the subject are separated by a pause period (760) in the case of detecting a positive response of the subject to the first presentation of the two successive presentations, and in particular also in the case of detecting a negative response of the subject to the first presentation of the two successive presentations
and/or wherein, when a late release of a response input means (140) to the presentation of an acoustic stimulus is detected,, a time interval (803) is added at the end of a pause period (760) after the presentation of the test point (t_{s,f}).

10. The computer-implemented method according to any of the preceding claims, wherein a positive response of the subject to the presentation of a test point (t_{s,f}) is registered as positive only if the response is detected within a predetermined time window (t_{R}) measured from the end of a predetermined reaction time period (773f) starting from the moment that the test point (t_{s,f}) is first presented to the subject at an amplitude level equal to a target level (703), and as falsely positive, if the positive response is detected outside the predetermined time window (t_{R}).

11. The computer-implemented method according to any of the preceding claims, wherein, when a positive response of the subject to the presentation of a test point (t_{s,f}) is providable by exerting a force, in particular a pressing force, on a response input means (140), which comprises force measuring means for measuring the exerted force, the method comprises receiving a signal representing a force level of the exerted force for providing the positive response to the presentation of the test point (t_{s,f}).

12. A data processing system (120; 110), comprising means configured to perform the method according to any of claims 1 to 11.

13. An audiometric system (100) comprising a data processing system (120; 110) according to claim 12,
wherein the audiometric system (100) preferably further comprises a sound reproducing means (130) for presenting an acoustic stimulus to a subject and/or a response input means (140), via which a response of the subject to the presented acoustic stimulus is providable to the audiometric system (100),
and/or
the audiometric system (100) is formed as an audiometer and/or comprises a server (110) and a client (120).

14. A computer program comprising instructions which, when the program is executed by a computer, cause the computer to carry out the method according to any of claims 1 to 11.

15. A computer-readable medium having stored thereon the computer program of claim 14.
